# EUROPÄISCHE PATENTSCHRIFT

(11) **EP 2 847 183 B1**
(45) Veröffentlichungstag und Bekanntmachung des Hinweises auf die Patenterteilung: **21.06.2017**
(21) Anmeldenummer: 13720943.3
(22) Anmeldetag: 06.05.2013
(51) Int. Cl.: C07D 403/14, C07D 249/04

(54) **VERFAHREN ZUR HERSTELLUNG VON TRIAZOL-VERBINDUNGEN**
PROCESS FOR THE PREPARATION OF TRIAZOLE COMPOUNDS
PROCEDE POUR LA PREPARATION DE COMPOSES TRIAZOLIQUES

(30) Priorität: 08.05.2012 EP 12167152
(43) Veröffentlichungstag der Anmeldung: 18.03.2015
(73) Patentinhaber: Bayer Pharma Aktiengesellschaft, 13353 Berlin (DE)
(72) Erfinder: MILITZER, Hans-Christian, 51519 Odenthal (DE); EGGERT, Johannes, 61250 Usingen (DE)
(74) Vertreter: BIP Patents
(86) Internationale Anmeldenummer: PCT/EP2013/059418
(87) Internationale Veröffentlichungsnummer: WO 2013/167552

(56) Entgegenhaltungen:
- WO-A1-03/101442
- WO-A1-2005/044785
- WO-A1-2008/067871

## Beschreibung

Die vorliegende Erfindung betrifft ein Verfahren zur Herstellung von 1-[6-(Morpholin-4-yl)pyrimidin-4-yl]-4-(1H-1,2,3-triazol-1-yl)-1H-pyrazol-5-ol (I - Enol-Form) bzw. 2-[6-(Morpholin-4-yl)pyrimidin-4-yl]-4-(1H-1,2,3-triazol-1-yl)-1,2-dihydro-3H-pyrazol-3-on (I - Keto-Form) und Natrium-1-[6-(morpholin-4-yl)pyrimidin-4-yl]-4-(1H-1,2,3-triazol-1-yl)-1H-pyrazol-5-olat (II) ausgehend von 1,2,3-Triazol (III), Bromessigsäuremethylester (IV-Me-Br) oder Bromessigsäureethylester (IV-Et-Br), 4,6-Dichlorpyrimidin (VIII), Morpholin (IX) und Hydrazin (XII).

Die Verbindung 1-[6-(Morpholin-4-yl)pyrimidin-4-yl]-4-(1H-1,2,3-triazol-1-yl)-1H-pyrazol-5-ol (Enol-Form) bzw. 2-[6-(Morpholin-4-yl)pyrimidin-4-yl]-4-(1H-1,2,3-triazol-1-yl)-1,2-dihydro-3H-pyrazol-3-on (Keto-Form) ist aus WO 2008/067871 bekannt und entspricht der Formel (I)

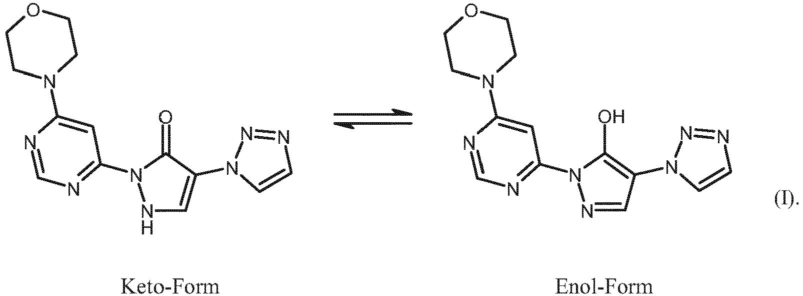

Die Verbindung Natrium-1-[6-(morpholin-4-yl)pyrimidin-4-yl]-4-(1H-1,2,3-triazol-1-yl)-1H-pyrazol-5-olat entspricht der Formel (II)

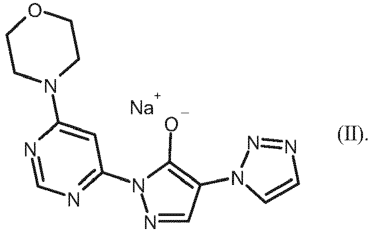

Die Verbindungen der Formeln (I) und (II) wirken als Hemmstoff der HIF-Prolyl-4-Hydroxylasen und führen aufgrund dieses spezifischen Wirkmechanismus *in vivo* nach parenteraler oder oraler Verabreichung die Induktion von HIF-Zielgenen, wie z.B. Erythropoetin, und der hierdurch verursachten biologischen Prozesse, wie z.B. Erythropoese, herbei.

In WO 2008/067871 ist eine Synthese zur Herstellung der Verbindung der Formel (I) im Gramm-Bereich, ausgehend von 1,2,3-Triazol (III), Bromessigsäureethylester (IV-Et-Br), 4,6-Dichlorpyrimidin (VIII), Morpholin (IX) und Hydrazin (XII) beschrieben:

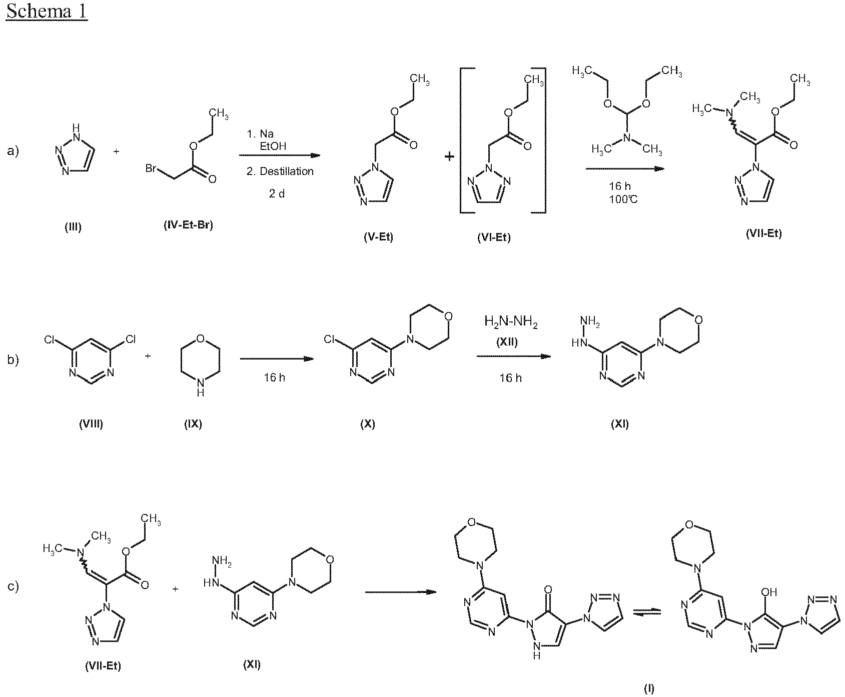

Diese Synthese der Verbindung der Formel (I) lässt sich in drei Abschnitte gliedern:
a) Die Herstellung der Verbindung der Formel (VII-Et) ausgehend von 1,2,3-Triazol (III) und Bromessigsäureethylester (IV-Et-Br) über die Verbindung der Formel (V-Et).
b) Die Herstellung der Verbindung der Formel (XI) ausgehend von 4,6-Dichlorpyrimidin (VIII), Morpholin (IX) und Hydrazin (XII).
c) Die Herstellung der Verbindung der Formel (I) durch Reaktion der Verbindungen der Formeln (VII-Et) und (XI).

### Stufe a)

Für eine technische Durchführung und die Produktion größerer kg-Mengen sind die in WO 2008/067871 beschriebenen Herstellprozesse nur mit Einschränkungen geeignet. So werden bei der Alkylierung von 1,2,3-Triazol (III) mit Bromessigsäureethylester (IV-Et-Br) mit Natriumethylat in Ethanol neben der gewünschten Verbindung der Formel (V-Et) auch etwa 30 bis 40% der isomeren Verbindung der Formel (VI-Et) gebildet. Die gewünschte Verbindung der Formel (V-Et) wird daher durch Vakuumdestillation von der isomeren Verbindung der Formel (VI-Et) getrennt. Zum einen führt die geringe Selektivität zu einer niedrigen Gesamtausbeute (50%), zum anderen wird die Destillation im Hochvakuum und nahe an den Zersetzungspunkten der Verbindungen der Formeln (V-Et) und (VI-Et) durchgeführt, und stellt daher im technischen Maßstab ein Sicherheitsrisiko dar. Die Reaktionszeit der Alkylierung von 2 Tagen ist aus technischer Sicht sehr lang, da dadurch teure technische Anlagenteile belegt und die Kosten der Herstellung erhöht werden. Für die Herstellung von Ethyl-3-(dimethylamino)-2-(1H-1,2,3-triazol-1-yl)acrylat (VII-Et) wird ebenfalls eine lange Reaktionszeit von 16 h benötigt. Über beide Stufen wird lediglich eine Ausbeute von 50% erreicht. Die Aufreinigung der Verbindung der Formel (VII-Et) kann durch Chromatographie wie in WO 2008/067871 beschrieben oder durch Verrühren mit Diethylether wie in DE 102008020113 beschrieben erfolgen. Die Chromatographie bedeutet im technischen Maßstab einen sehr hohen apparativen Aufwand, der mit entsprechenden zusätzlichen Kosten verbunden ist, und das Verrühren mit Diethylether ist mit weiterem Ausbeuteverlust beschrieben. Insgesamt stellt die geringe Selektivität der Alkylierung des 1,2,3-Triazols (III) mit Bromessigsäureethylester (IV-Et-Br) den entscheidenden Nachteil dieses Verfahrens dar und trägt ganz wesentlich zu der niedrigen Ausbeute von maximal 50% bei.

Es ist bekannt, dass Alkylierungen von 1,2,3-Triazol (III) üblicherweise nur mit einer geringen Selektivität zu Gunsten des 1-substituierten Produktes verlaufen. Nach H. Gold, Liebigs Annalen der Chemie (1965) 205 ff, lässt sich 1,2,3-Triazol (III) mit Alkylhalogeniden nur mit geringen Selektivitäten alkylieren. Typischerweise werden in den jeweiligen Produkten Zusammensetzungen der 1- und 2-substituierten Triazole von etwa 3:2 bis maximal 4:1 erhalten. Für die Alkylierung von 1,2,3-Triazol (III) mit Bromessigsäureethylester (IV-Et-Br) in Gegenwart von Natriummethylat wurde ein Verhältnis von 3:2 gefunden (Beispiel 10 und 11 in H. Gold). Wegen der annähernden Gleichwertigkeit der Stickstoffatome im 1,2,3-Triazol (III) wäre ein Mengenverhältnis von 2:1 (N-1-Isomer zu N-2-Isomer) zu erwarten gewesen, das zu den experimentell gefundenen Werten recht gut passt. Höhere Anteile an dem hier gewünschten N-1-Isomer wurde von H. Gold bei der Alkylierung von 1,2,3-Triazol (III) mit Propylbromid und Allylbromid erzielt, wenn Triazol im Überschuss eingesetzt wurde. Allerdings sind dabei die erzielten Umsätze sehr unvollständig, die Ausbeuten gering und die Reaktionszeiten sehr lang.

In WO 2006/114706, WO 2006/123242 und US 20050154024 wird ebenfalls die Alkylierung von 1,2,3-Triazol (III) mit Bromessigsäureethylester (IV-Et-Br) beschrieben. Bei der dabei gewählten Durchführung mit Kaliumcarbonat in Ethanol wurde allerdings nur eine sehr geringe Selektivität von 5:6 (N-1-Isomer zu N-2-Isomer) beobachtet. Die gleiche Umsetzung mit Natriumcarbonat als Base in Aceton als Lösungsmittel wird von M. Kume, J. Antibiot. 46 (1993) 177 beschrieben. Nach 5 Tagen Reaktionszeit bei 30°C und Aufreinigung mittels Chromatographie wurden 65% der Verbindung der Formel (V-Et) und 26% der Verbindung der Formel (VI-Et) erhalten, was etwa einem Isomerenverhältnis von 2,5:1 gleich kommt.

Ethyl-1H-1,2,3-triazol-1-yl-acetat (V-Et) kann alternativ auch durch eine [3+2]-Cycloaddition von Acetylen (XIII) und 2-Azidoessigsäureethylester (XIV-Et) hergestellt werden, bei der nur das gewünschte Regioisomer der Formel (V-Et) entsteht. Die Ausbeute für diese Reaktion wird von L. Fisera und D. Pavlovic, Collection of Czech. Chem. Commun. 49 (1984) 1990 allerdings mit nur 11% berichtet. Eine Ausbeute von 74% wird von B. Rickborn, Organic Reactions 52 (1998) berichtet. Acetylen (XIII) sowie insbesondere 2-Azidoessigsäureethylester (XIV-Et) sind sehr energiereiche Verbindungen. Organische Azide zerfallen bereits bei Zufuhr geringer Energiemengen, wie z.B. durch Schlag sowie Druck- oder Temperaturerhöhung, unter Stickstoffentwicklung. Sie können sich dabei explosionsartig zersetzen. Für eine Reaktion von Acetylen (XIII) mit 2-Azidoessigsäureethylester (XIV-Et) im technischen Maßstab unter Druck sind daher besonderes hohe Sicherheitsanforderungen zu erfüllen und es werden geeignete Autoklaven benötigt. Diese Anforderungen werden nur von sehr wenigen technischen Anlagen erfüllt.

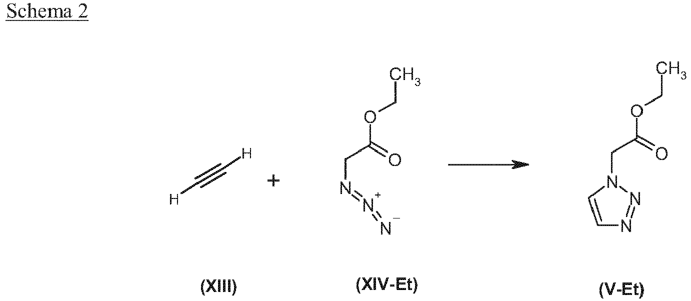

### Stufe b)

Bei der in WO 2008/067871 beschriebenen Herstellung von 4-(6-Hydrazinopyrimidin-4-yl)morpholin (XI) ausgehend von 4,6-Dichlorpyrimidin (VIII), Morpholin (IX) und 10 Moläquivalenten Hydrazinhydrat (XII-Hydrat) wird eine Ausbeute von 58% über beide Stufen nach Reaktionszeiten von jeweils 16 h erreicht. Für eine technische Umsetzung sind die geringe Ausbeute und die langen Reaktionszeiten unbefriedigend. Die Verwendung des großen Überschusses an 10 Moläquivalenten des im Tierversuch krebserregenden und giftigen Hydrazinhydrates (XII-Hydrat) ist für eine technische Umsetzung ungeeignet, da sie eine aufwändige Abwasserbehandlung erfordert und das Produkt durch einen Hydrazingehalt oberhalb von 100 ppm belastet ist. In WO 2004/046120 wird der gleiche Syntheseweg zu 4-(6-Hydrazinopyrimidin-4-yl)morpholin (XI) gewählt, allerdings sind keine Details zur Herstellung angegeben. In WO 2003/101442 wird ebenfalls der gleiche Syntheseweg angewendet, allerdings werden im zweiten Reaktionsschritt unter Mikrowellenbeheizung bei 120°C 3 Moläquivalente Hydrazinhydrat (XII-Hydrat) zur Reaktion gebracht. Die Verbindung der Formel (XI) wird durch präparative Chromatographie aufgereinigt. Eine Mikrowellenbeheizung ist im technischen Maßstab derzeit nicht praktikabel, die erforderliche chromatographische Aufreinigung würden im technischen Maßstab einen zusätzlichen hohen Aufwand bedeuten.

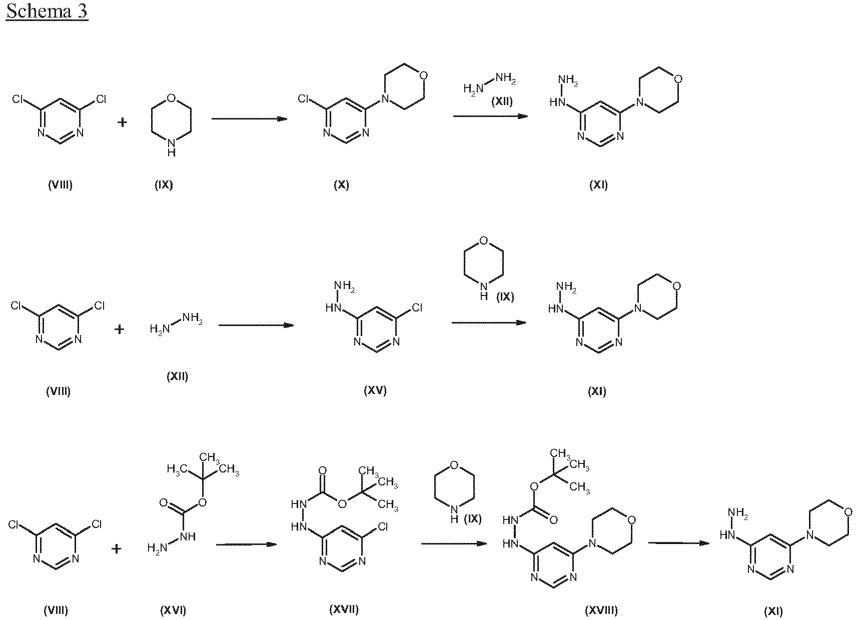

In EP 121341 wird die Herstellung der Verbindung der Formel (XI) durch Umsetzung der Verbindung der Formel (XV) mit 2 Moläquivalenten Morpholin (IX) in Wasser für 16 h bei Rückflussbedingungen, nachfolgende Extraktion der Verbindung der Formel (XI) mit Chloroform und Isolierung durch Einengen des Extraktes beschrieben. Die Extraktion mit Chloroform sowie die Gewinnung des Produktes durch Einengen des Extraktes sind für eine technische Umsetzung nur sehr bedingt geeignet, da Chloroform im Verdacht steht, krebserregend zu sein.

Postovskii, Smirnova und Kirov, Polytech. Inst., Sverdlovsk, Doklady Akademii Nauk SSSR (1966), 166(5), 1136-9 beschreiben ebenfalls die zweistufige Herstellung der Verbindung der Formel (XI) über die Zwischenverbindung der Formel (XV). Die erzielte Ausbeute von weniger als 50% über beide Schritte ist allerdings unbefriedigend.

Bei Sicherheitsuntersuchungen an der Verbindung der Formel (XV) zeigte sich, dass diese Verbindung sehr energiereich ist und als pulverförmiger Feststoff einer Deflagration unterliegen kann. Die Möglichkeit einer Deflagration stellt für den Umgang mit diesem Material insbesondere im technischen Maßstab ein Sicherheitsrisiko dar und bedingt zusätzliche technische Sicherheitsvorkehrungen, sofern dieses Material in trockener Form in Behältern mit beweglichen Einbauten, wie z.B. Trocknungsapparaturen mit mechanischer Rührung, gehandhabt werden soll, da im Falle von Reibung z. B. bei einer Wandberührung des Rührers ausreichende Temperaturen für eine Zündung entstehen können. Für eine Herstellung im technischen Maßstab sind derartige Apparate allerdings bevorzugt, da sie eine geschlossene Handhabung auch größerer Mengen trockenen Produktes bis zur Abfüllung erlauben, ohne dass Mitarbeiter in Kontakt mit dem Produkt kommen können.

In WO 2003/101442 wird die Herstellung der Verbindung der Formel (XI) über die Umsetzung der Verbindung der Formel (VIII) mit tert-Butylcarbazat (XVI) bei 120°C mit Mikrowellenbeheizung zu der Zwischenverbindung der Formel (XVII) beschrieben. Die Verbindung der Formel (XVII) wird mit Morpholin (IX) zu einer weiteren Zwischenverbindung der Formel (XVIII) umgesetzt, aus der anschließend durch Abspaltung der Schutzgruppe und präparativer Chromatographie die Verbindung der Formel (XI) mit einer Gesamtausbeute von 50% erhalten wird. Erneut sprechen sowohl Mikrowellenbeheizung als auch die erforderliche chromatographische Aufreinigung gegen eine technische Umsetzung dieses Verfahrens.

### Stufe c)

Die in WO 2008/067871 für die Herstellung der Verbindung der Formel (I) aus den Verbindungen der Formeln (VII-Et) und (XI) beschriebene Ausbeute von 61% nach 16 h Reaktionszeit in siedendem Essigsäureethylester und in Gegenwart von Trifluoressigsäure ist für ein technisches Verfahren unbefriedigend. Darüber hinaus enthält das aus Essigsäureethylester isolierte Produkt eingeschlossene Salze der Trifluoressigsäure und entspricht damit nicht den Anforderungen, die hinsichtlich der Reinheit an einen pharmazeutischen Wirkstoff angelegt werden.

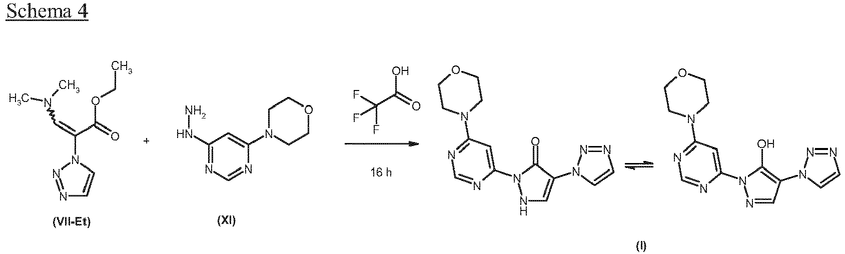

Eine Synthese der Verbindung der Formel (II) wurde bislang noch nicht beschrieben. Da die Verbindung der Formel (II) das Natriumsalz der Verbindung der Formel (I) ist, sollte eine Herstellung der Verbindung der Formel (II) durch Umsetzung der Verbindung der Formel (I) mit einem basischen Natriumsalz, wie zum Beispiel Natriumhydroxid, gelingen können:

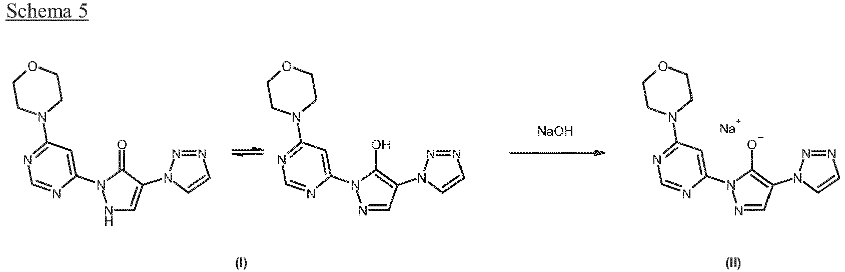

Daraus ergibt sich die Aufgabe der vorliegenden Erfindung, ein Verfahren zur Herstellung der Verbindung der Formel (I) sowie der Verbindung der Formel (II) bereitzustellen, das insbesondere für die Herstellung größerer Produkt-Mengen in guter Ausbeute und mit hoher Reinheit geeignet ist.

### Stufe a)

Überraschend wurde nun gefunden, dass die Alkylierung von 1,2,3-Triazol (III) mit Bromessigsäurealkylester (IV-R-Br; R = Methyl (Me), Ethyl (Et)) durch die Verwendung von Ethyldiisopropylamin als Base zu Selektivitäten von ≥ 6:1 zu Gunsten des gewünschten Alkyl-1H-1,2,3-triazol-1-ylacetat (V-R; R = Methyl (Me), Ethyl (Et)) bezogen auf das Alkyl-2H-1,2,3-triazol-2-ylacetat (VI-R; R = Methyl (Me), Ethyl (Et)) führt, ohne dass dabei große Überschüsse des Alkylierungsmittels erforderlich sind oder nur niedrige Reaktionsumsätze erzielt werden. Dieses Maß an Selektivität liegt deutlich höher als die bislang für Alkylierungen von 1,2,3-Triazol (III) berichteten Werte. Insbesondere ermöglicht die höhere Selektivität der Alkylierung auch eine höhere Gesamtausbeute bei der Herstellung der Verbindung der Formel (I) bzw. Verbindung der Formel (II) bezogen auf 1,2,3-Triazol (III). Die Umsetzung erfolgt im Allgemeinen in inerten Lösungsmitteln.

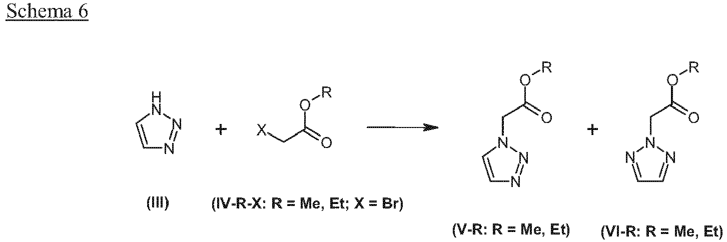

Bevorzugt wird die Umsetzung mit Bromessigsäuremethylester (IV-Me-Br) oder Bromessigsäureethylester (IV-Et-Br), ganz besonders bevorzugt mit Bromessigsäuremethylester (IV-Me-Br) durchgeführt. Bevorzugt wird die Umsetzung in einem Essigsäurealkylester eines kurzkettigen Alkohols (C-1 bis C-4) als Lösungsmittel, ganz besonders bevorzugt in Essigsäureethylester als Lösungsmittel durchgeführt. Besonders bevorzugt werden 0,9 bis 1,8 Moläquivalente, ganz besonders bevorzugt werden 1,1 bis 1,3 Moläquivalente des Bromessigsäurealkylester (IV-R-Br) bezogen auf 1,2,3-Triazol (III) eingesetzt. Bevorzugt wird die Reaktion bei 20 bis 80°C, besonders bevorzugt bei 30 bis 50°C, ganz besonders bevorzugt bei 35 bis 45°C durchgeführt. Besonders bevorzugt werden 1,2 bis 3 Moläquivalente, ganz besonders bevorzugt werden 1,8 bis 2,2 Moläquivalente Ethyldiisopropylamin eingesetzt. Bevorzugt wird der Bromessigsäurealkylester (IV-R-Br) über 0,5 bis 16 h zu einer gerührten Mischung aus 1,2,3-Triazol (III) und Ethyldiisopropylamin in einem Lösungsmittel zudosiert, ganz besonders bevorzugt wird diese Dosierung bei einer Temperatur von 30 bis 40°C durchgeführt. Bevorzugt wird nach der Dosierung des Bromessigsäurealkylesters (IV-R-Br) die Reaktionsmischung noch für 2 bis 24 h bei 30 bis 50°C nachgerührt, ganz besonders bevorzugt wird die Reaktionsmischung für 4 bis 10 h bei 35 bis 45°C nachgerührt.

In einer Variante des Verfahrens werden bevorzugt 0,05 bis 0,5 Moläquivalente bzw. ganz besonders bevorzugt 0,1 bis 0,3 Moläquivalente Ethyldiisopropylamin in Gegenwart von 1,0 bis 2,0 Moläquivalenten Natriumhydrogencarbonat als Base eingesetzt.

Die in der Reaktion gebildeten Salze können durch Filtration oder Lösen in Wasser von den Produkten entfernt werden. Bevorzugt ist die Abtrennung der Salze durch Filtration des Reaktionsgemisches über ein geeignetes Filteraggregat. Besonders bevorzugt wird diese Filtration bei einer Temperatur von -5 bis 25 °C durchgeführt.

Die so erhaltene Lösung des Produktgemisches kann durch Destillation aufkonzentriert und z. B. durch Kristallisation aufgereinigt werden. Alternativ und bevorzugt wird die Lösung des Produktgemisches der Verbindungen der Formeln (V-R) und (VI-R) ohne weitere Aufreinigung in die Umsetzung zu der Verbindung der Formel (VII-R) eingesetzt. Ganz besonders bevorzugt wird vor der Umsetzung zu der Verbindung der Formel (VII-R) ein Teil des Lösungsmittels durch Destillation entfernt.

Weiterhin wurde gefunden, dass bei der Alkylierung von 1,2,3-Triazol (III) mit Bromessigsäurealkylester (IV-R-Br; R = Methyl (Me), Ethyl (Et)) auch unter Verwendung von 1,5 bis 3 Äquivalenten Natriumhydrogencarbonat in Acetonitril hohe Selektivitäten von ≥ 6:1 erreicht werden. Die Reaktion wird bevorzugt in einem Temperaturbereich von 30 bis 90 °C, besonders bevorzugt in einem Temperaturbereich von 50 bis 70 °C und einer Reaktionszeiten von 16 bis 48 h, besonders bevorzugt einer Reaktionszeiten von 20 bis 30 h, durchgeführt.

Überraschendweise wurde gefunden, dass die Verbindungen der Formeln (VII-Me) bzw. (VII-Et) in einem einfachen Verfahren auch in größeren Mengen und hoher Produktqualität erhalten werden können, in dem eine Lösung der Rohprodukte aus der Herstellung der Verbindungen der Formeln (V-Me) und (VI-Me) bzw. Verbindungen der Formeln (V-Et) und (VI-Et), die in einem Verhältnis von mindestens 6:1 zueinander vorliegen, mit Dimethylformamid-Dimethylacetal (XIX-Me) bzw. Dimethylformamid-Diethylacetal (XIX-Et) in einem ersten inerten Lösungsmittel bei erhöhter Temperatur umgesetzt wird. Das jeweilige Produkt der Verbindung der Formel (VII-Me) bzw. der Verbindung der Formel (VII-Et) wird anschließend durch Abkühlen der Lösung oder durch Abdestillation des Lösungsmittels und Zugabe eines zweiten Lösungsmittels kristallisiert. Gegebenenfalls wird vor der Kristallisation ein drittes Lösungsmittel zugegeben, die Lösung filtriert und das Lösungsmittel abdestilliert.

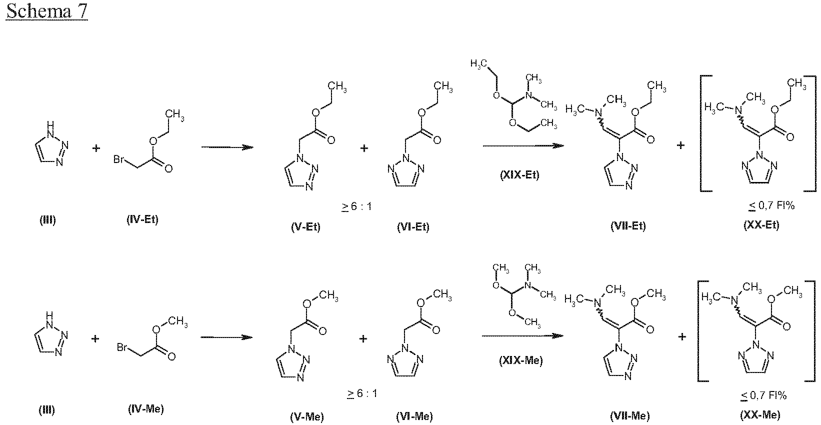

Bevorzugt wird die Umsetzung mit einer Lösung der Rohprodukte aus der Herstellung der Verbindungen der Formeln (V-Me) und (VI-Me), die in einem Verhältnis von mindestens 6:1 zueinander vorliegen, mit Dimethylformamid-Dimethylacetal (XIX-Me) durchgeführt.

Bevorzugt wird die Umsetzung in einem ersten inerten Lösungsmittel durchgeführt. Besonders bevorzugt wird die Reaktion in einem Essigsäurealkylester oder Alkylalkohol durchgeführt, bei dem Alkyl für Methyl, Ethyl, 1-Propyl, 1- Butyl, 2-Propyl, 2-Butyl steht. Alternativ kann Dimethylformamid-Dimethylacetal (XIX-Me) bzw. Dimethylformamid-Diethylacetal (XIX-Et) als Lösungsmittel und Reaktionspartner dienen.

Bezogen auf die in die jeweilige Alkylierung eingesetzte Menge an 1,2,3-Triazol (III) werden 0,9 bis 4,0 Moläquivalente Dimethylformamid-Dimethylacetal (XIX-Me) bzw. Dimethylformamid-Diethylacetal (XIX-Et) eingesetzt. Bevorzugt werden 1,1 bis 3,0 Moläquivalente, ganz besonders bevorzugt werden 1,2 bis 2,0 Moläquivalente Dimethylformamid-Dimethylacetal (XIX-Me) bzw. Dimethylformamid-Diethylacetal (XIX-Et) eingesetzt.

Die Reaktion wird in einem Temperaturbereich von 60 bis 120°C durchgeführt. Bevorzugt wird sie bei 70 bis 90°C durchgeführt. Bevorzugt wird das Reaktionsgemisch 0,5 bis 8 h bei der Reaktionstemperatur gerührt. Besonders bevorzugt wird das Reaktionsgemisch für 1 bis 6 h bei 70 bis 90°C gerührt. Ganz besonders bevorzugt wird das Reaktionsgemisch für 2 bis 4 h bei 75 bis 90°C gerührt.

Bevorzugt werden bei der Reaktion entstehende Leichtsieder abdestillieren.

Bevorzugt wird zu dem Reaktionsgemisch nach der Reaktion eine drittes Lösungsmittels wie Aceton, Essigsäureethylester oder Tetrahydrofuran gegeben und die Lösung filtriert, um in der Reaktion gebildete feste Verunreinigungen zu entfernen. Besonders bevorzugt wird die Filtration bei einer Temperatur von 50 bis 80°C durchgeführt.

Bevorzugt werden die Verbindungen der Formeln (VII-Me) bzw. (VII-Et) aus der zuvor filtrierten Reaktionsmischung durch Abkühlung auf eine Temperatur von -5 bis 25 °C der Lösung kristallisiert und die Kristalle durch nachfolgende Filtration isoliert. Die Kristallisation erfolgt bevorzugt aus einem zweiten Lösungsmittel wie Isopropanol, Essigsäureethylester, Aceton oder Methyl-tert-butylether oder einer geeigneten Mischung dieser Lösungsmittel. Besonders bevorzugt erfolgt die Kristallisation aus Isopropanol. Sofern die Kristallisation in einem anderen Lösungsmittel als dem der Reaktion durchgeführt wird, wird der Wechsel des Lösungsmittels bevorzugt durch Destillation durchgeführt.

Die Qualität der so hergestellten Verbindungen ist ausreichend hoch, um ohne chromatographische Aufreinigungsprozesse in die Herstellung der Verbindung der Formel (I) bzw. der Verbindung der Formel (II) eingesetzt werden zu können. Insbesondere zeichnet sich die Qualität durch einen niedrigen Gehalt der isomeren Verunreinigungen der Verbindung der Formel (XX-Me) bzw. der Verbindung der Formel (XX-Et) aus. Das Erreichen eines möglichst niedrigen Gehaltes der jeweils isomeren Verunreinigungen der Verbindung der Formel (XX-Me) bzw. der Verbindung der Formel (XX-Et) mit einem einfachen Verfahren ohne mehrfache Aufreinigungsschritte ist eine wesentliche Voraussetzung dafür, dass die Verbindung der Formel (I) bzw. der Verbindung der Formel (II) die für einen pharmazeutischen Wirkstoff erforderliche Qualität auch bei der Herstellung größerer Mengen sicher erreichen kann, ohne dass technisch aufwändige Aufreinigungsoperation erforderlich werden.

### Stufe b)

Überraschend wurde zusätzlich gefunden, dass größere Mengen der Verbindung der Formel (XI) in einem einfachen Verfahren, mit guter Qualität und ohne zusätzliche Aufreinigungsschritte wie z.B. einer Chromatographie ausgehend von 4,6-Dichlorpyrimidin (VIII), Morpholin (IX) und nur ≤ 2 Moläquivalenten Hydrazinhydrat (XII-Hydrat) (bezogen auf 4,6-Dichlorpyrimidin (VIII)) herstellbar sind, wenn in der ersten Stufe zunächst 4,6-Dichlorpyrimidin (VIII) mit Hydrazinhydrat (XII-Hydrat) in einem Lösungsmittel, gegebenenfalls in Gegenwart einer Hilfsbase, umgesetzt und das erhaltene Reaktionsgemisch ohne Isolierung der gebildeten Verbindung der Formel (XV) in einer zweiten Stufe nach Zugabe von Morpholin (IX) und einer weiteren Hilfsbase erhitzt, sowie die Verbindung der Formel (XI) anschließend nach Kristallisation isoliert wird. Eine Handhabung der Verbindung der Formel (XV) in trockener, deflagrationsfähiger Form sowie hohe Überschüsse des in Tierversuchen krebserregenden und giftigen Hydrazinhydrates (XII-Hydrat) können so vermieden werden.

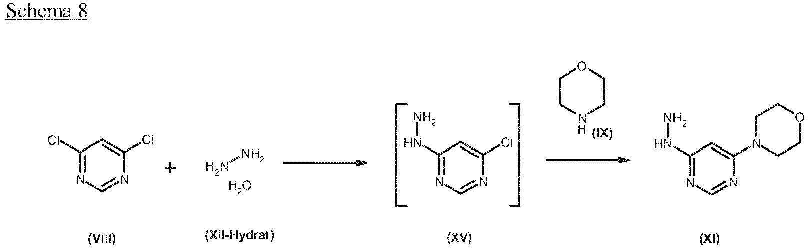

Bevorzugt wird die Reaktion in Wasser oder einem Alkylalkohol, bei dem Alkyl für Methyl, Ethyl, 1-Propyl, 1-Butyl, 2-Propyl, 2-Butyl steht, oder einer Mischung dieser Lösungsmittel durchgeführt.

Besonders bevorzugt wird die Reaktion in Wasser durchgeführt. Ganz besonders bevorzugt werden 2,4 bis 3,8 kg Wasser pro 1 kg 4,6-Dichlorpyrimidin (VIII) verwendet.

Bezogen auf 1 Äquivalent 4,6-Dichlorpyrimidin (VIII) werden in diesem Verfahren in der ersten Stufe bevorzugt 0,9 bis 2,0 Moläquivalente Hydrazinhydrat (XII-Hydrat) eingesetzt. Besonders bevorzugt werden 1,0 bis 1,5 Moläquivalente Hydrazinhydrat (XII-Hydrat) und 1,0 bis 1,5 Moläquivalente Triethylamin oder Ethyldiisopropylamin als Hilfsbase, ganz besonders bevorzugt werden 1,1 bis 1,3 Moläquivalente Hydrazinhydrat (XII-Hydrat) und 1,1 bis 1,3 Moläquivalente Triethylamin als Hilfsbase bezogen auf 1 Äquivalent 4,6-Dichlorpyrimidin (VIII) eingesetzt.

Bevorzugt erfolgt die Dosierung von Hydrazinhydrat (XII-Hydrat) zu in dem Lösungsmittel vorgelegtem 4,6-Dichlorpyrimidin (VIII) und der gegebenenfalls verwendeten Hilfsbase bei 0 bis 25°C, besonders bevorzugt erfolgt diese Dosierung bei 10 bis 20°C. In einer Variante des Verfahrens erfolgt die Dosierung von Triethylamin oder Ethyldiisopropylamin zu in dem Lösungsmittel vorgelegtem 4,6-Dichlorpyrimidin (VIII) und Hydrazinhydrat (XII-Hydrat) bei 0 bis 25°C, besonders bevorzugt erfolgt die Dosierung von Triethylamin bei 10 bis 20°C.

Bevorzugt wird das so erzeugte Reaktionsgemisch der ersten Stufe bei einer Temperatur von 10 bis 30°C, besonders bevorzugt bei 20 bis 25°C gerührt. Bevorzugt dauert die Reaktionszeit der ersten Stufe 3 bis 24 h, besonders bevorzugt wird das Reaktionsgemisch 8 bis 16 h bei 20 bis 25°C gerührt.

Bevorzugt erfolgt keine Isolierung des auf diese Weise erzeugten 4-Chlor-6-hydrazinopyrimidins (XV). Besonders bevorzugt wird die so erhaltene Lösung bzw. Suspension des 4-Chlor-6-hydrazinopyrimidins (XV) in der zweiten Stufe durch Zugabe von Morpholin (IX) direkt weiter zu 4-(6-Hydrazinopyrimidin-4-yl)morpholin (XI) umgesetzt.

Bevorzugt werden in der zweiten Stufe 0,9 bis 1,5 Moläquivalente Morpholin (IX) bezogen auf eingesetztes 4,6-Dichlorpyrimidin (VIII) der Reaktionsmischung aus der ersten Stufe zugegeben. Besonders bevorzugt werden 1,0 bis 1,3 Moläquivalente Morpholin (IX) bezogen auf eingesetztes 4,6-Dichlorpyrimidin (VIII) der Reaktionsmischung zugegeben.

Bevorzugt wird in der zweiten Stufe eine zusätzliche anorganische Hilfsbase, z.B. Natriumhydrogencarbonat, Kaliumhydrogencarbonat, Natriumhydroxid oder Kaliumhydroxid, bei der Umsetzung mit Morpholin (IX) verwendet. Besonders bevorzugt werden 1,0 bis 2,0 Moläquivalente Natriumhydrogencarbonat, ganz besonders bevorzugt werden 1,1 bis 1,3 Moläquivalente Natriumhydrogencarbonat verwendet.

Zur Bildung von 4-(6-Hydrazinopyrimidin-4-yl)morpholin (XI) wird die Morpholin (IX) enthaltene Reaktionsmischung der zweiten Stufe bevorzugt bei einer Temperatur von 70 bis 110°C und besonders bevorzugt bei 75 bis 95°C gerührt. Die Reaktionszeit beträgt bevorzugt 4 bis 24 h. Besonders bevorzugt wird die Reaktionsmischung bei 75 bis 95°C für 6 bis 10 h gerührt.

Bevorzugt wird 4-(6-Hydrazinopyrimidin-4-yl)morpholin (XI) nach der Reaktion durch Kristallisation und Filtration isoliert. Besonders bevorzugt erfolgt die Filtration bei einer Temperatur von 5 bis 25°C. Bevorzugt wird die erhaltene Verbindung der Formel (XI) mit Wasser gewaschen.

### Stufe c)

Darüber hinaus wurde überraschend gefunden, dass die Verbindung der Formel (I) in größeren Mengen mit guter Ausbeute und sehr guter Qualität nach einem einfachen, technisch durchführbaren Verfahren hergestellt werden kann.

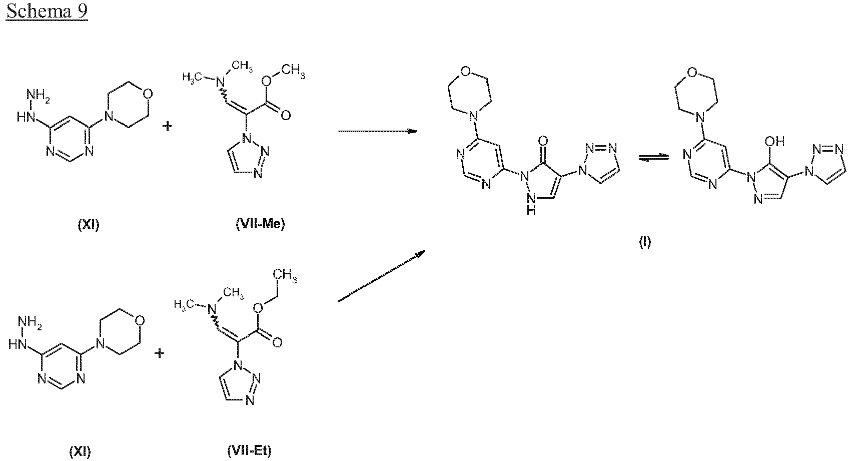

Bevorzugt werden dafür 4-(6-Hydrazinopyrimidin-4-yl)morpholin (XI) und eine der Verbindungen der Formeln (VII-Me) oder (VII-Et) in Gegenwart einer Säure und in einem inerten Lösungsmittel umgesetzt. Besonders bevorzugt werden 4-(6-Hydrazinopyrimidin-4-yl)morpholin (XI) und Methyl-(2E/Z)-3-(dimethylamino)-2-(1H-1,2,3-triazol-1-yl)acrylat (VII-Me) in Gegenwart einer Säure und in einem inerten Lösungsmittel umgesetzt.

Bevorzugt wird als Lösungsmittel ein Alkylester der Ameisensäure, Essigsäure oder Propionsäure, ein Alkylalkohol oder ein Alkylcyanid, wobei Alkyl für Methyl, Ethyl, 1-Propyl, 1-Butyl, 2-Propyl, 2-Butyl steht, oder eine geeignete Mischung dieser Lösungsmittel verwendet. Besonders bevorzugt wird als Lösungsmittel Essigsäureethylester, Essigsäure-n-butylester oder n-Butyronitril verwendet.

Bevorzugt wird Trifluoressigsäure als Säure verwendet. Besonders bevorzugt werden 0,2 bis 1,0 Moläquivalente Trifluoressigsäure im Verhältnis zu 4-(6-Hydrazinopyrimidin-4-yl)morpholin (XI) eingesetzt. Ganz besonders bevorzugt werden 0,4 bis 0,6 Moläquivalente Trifluoressigsäure im Verhältnis zu 4-(6-Hydrazinopyrimidin-4-yl)morpholin (XI) eingesetzt.

Bevorzugt werden von den Verbindungen der Formeln (VII-Me) oder (VII-Et) 0,9 bis 1,5 Moläquivalente im Verhältnis zu 4-(6-Hydrazinopyrimidin-4-yl)morpholin (XI) eingesetzt. Besonders bevorzugt werden von den Verbindungen der Formeln (VII-Me) oder (VII-Et) 1,0 bis 1,2 Moläquivalente im Verhältnis zu 4-(6-Hydrazinopyrimidin-4-yl)morpholin (XI) eingesetzt.

Bevorzugt wird die Reaktion in einem Temperaturbereich von 70 bis 140°C durchgeführt. Besonders bevorzugt wird die Reaktion in einem Temperaturbereich von 75 bis 120°C durchgeführt.

Bevorzugt beträgt die Reaktionszeit in Essigsäureethylester 20 bis 30 h bei 75 bis 90°C und die Reaktionszeit in Essigsäure-n-butylester oder n-Butyronitril beträgt 5 bis 10 h bei 110 bis 120°C.

Bevorzugt wird die Reaktionsmischung zur Isolierung eines Rohproduktes der Formel (I) auf 0 bis 25°C abgekühlt und filtriert. Besonders bevorzugt erfolgt diese Filtration bei 0 bis 10°C.

Bevorzugt wird das erhaltene Rohprodukt der Formel (I) zur Aufreinigung mit Wasser und einer Säure gemischt und die erhalten Mischung wird erneut filtriert. Besonders bevorzugt erfolgt die Aufreinigung mit Wasser und einer Säure bei einem pH-Wert von 4 bis 5,5. Ganz besonders bevorzugt erfolgt die Aufreinigung mit Wasser und Essigsäure bei einem pH-Wert von 4 bis 5,5.

Bevorzugt wird das durch Filtration isolierte Produkt der Formel (I) im Vakuum getrocknet.

In einer Variante des Verfahrens zur Herstellung der Verbindung der Formel (I) wird die Reaktionsmischung bestehend aus den Verbindungen der Formeln (VII-Me) oder (VII-Et) und 4-(6-Hydrazinopyrimidin-4-yl)morpholin (XI) sowie Trifluoressigsäure in einem Lösungsmittel nach einer ersten Reaktionszeit in einem Temperaturbereich von 60 bis 120°C, bevorzugt 70 bis 90°C, mit einer Base versetzt. Bevorzugt werden bei dieser Variante des Verfahrens 1,0 bis 1,5 Moläquivalente Trifluoressigsäure im Verhältnis zu 4-(6-Hydrazinopyrimidin-4-yl)morpholin (XI) eingesetzt. Bevorzugt beträgt die erste Reaktionszeit vor Zugabe der Base 2 bis 5 h. Bevorzugt wird Essigsäureethylester als Lösungsmittel verwendet.

Bevorzugt wird bei dieser Variante des Verfahrens als Base ein tertiäres Amin eingesetzt. Ganz besonders bevorzugt wird als Base Triethylamin eingesetzt.

Bevorzugt werden bei dieser Variante des Verfahrens 1,5 bis 3 Moläquivalente der Base bezogen auf eingesetztes 4-(6-Hydrazinopyrimidin-4-yl)morpholin (XI) verwendet. Besonders bevorzugt werden 1,8 bis 2,7 Moläquivalente der Base bezogen auf eingesetztes 4-(6-Hydrazinopyrimidin-4-yl)morpholin (XI) verwendet.

Bevorzugt wird bei dieser Variante des Verfahrens das Reaktionsgemisch nach Zugabe der Base bei einer Temperatur von 20 bis 90°C, besonders bevorzugt bei einer Temperatur von 50 bis 80°C gehalten.

Bevorzugt beträgt bei dieser Variante des Verfahrens die Reaktionszeit nach Zugabe der Base 1 bis 12 h, ganz besonders bevorzugt beträgt die Reaktionszeit 2 bis 5 h.

Bevorzugt wird bei dieser Variante des Verfahrens die Reaktionsmischung zur Isolierung eines Rohproduktes der Formel (I) auf -10 bis 25°C abgekühlt und filtriert. Besonders bevorzugt erfolgt diese Filtration bei 0 bis 10°C.

Bevorzugt wird das erhaltene Rohprodukt der Verbindung der Formel (I) zur Aufreinigung mit Wasser und einer Säure gemischt und die erhalten Mischung wird erneut filtriert. Besonders bevorzugt erfolgt die Aufreinigung mit Wasser und einer Säure bei einem pH-Wert von 4 bis 5,5. Ganz besonders bevorzugt erfolgt die Aufreinigung mit Wasser und Essigsäure bei einem pH-Wert von 4 bis 5,5.

Bevorzugt wird das durch Filtration isolierte Produkt der Formel (I) im Vakuum getrocknet.

Überraschend wurde gefunden, dass die Verbindung der Formel (II) in größeren Mengen mit sehr guten Ausbeuten und sehr guter Qualität in einem einfachen Verfahren aus der Verbindung der Formel (I) durch Umsetzung mit Natriumhydroxid oder wässriger Natronlauge oder Natriummethylat oder Natriumethylat oder einem Natrium-Salz hergestellt werden kann.

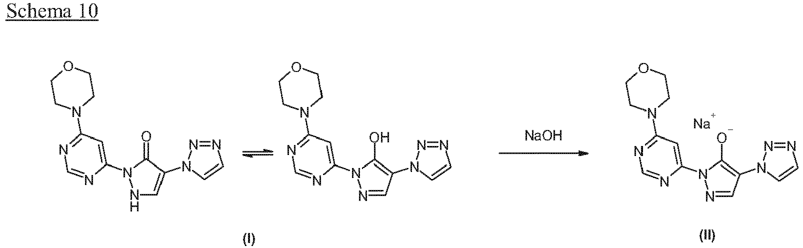

Bevorzugt wird die Verbindung der Formel (I) in der ersten Stufe zuerst in einem geeigneten Lösungsmittel durch Zugabe einer organischen Base gelöst und zur Abtrennung von gegebenenfalls vorhandenen, nichtlöslichen Bestandteile filtriert. Die gegebenenfalls filtrierte Lösung der Verbindung der Formel (I) wird in einer zweiten Stufe mit Natriumhydroxid oder wässriger Natronlauge oder Natriummethylat oder Natriumethylat oder einem Natrium-Salz umgesetzt.

Als Lösungsmittel eignen sich niedere Alkohole wie beispielsweise Methanol, Ethanol, n-Propanol, iso-Propanol, n-Butanol, sec-Butanol, iso-Butanol, 1-Pentanol, oder Tetrahydrofuran, oder Acetonitril, oder Aceton, oder Toluol, oder 1,4-Dioxan oder Gemische der genannten Lösungsmittel, oder Gemische der genannten Lösungsmittel mit Wasser. Bevorzugt sind Methanol, Ethanol, 2-Propanol, Tetrahydrofuran oder Gemische der genannten Lösungsmittel mit Wasser. Besonders bevorzugt sind Mischungen von Methanol oder Ethanol mit Wasser in einem Verhältnis zwischen 1:1 und 50:1 (v/v), ganz besonders bevorzugt sind Mischungen von Methanol mit Wasser in einem Verhältnis zwischen 7:3 und 30:1 (v/v).

Als organische Basen in der ersten Stufe eignen sich tertiäre Amine wie beispielsweise Triethylamin oder Diisopropylethylamin. Bevorzugt ist Triethylamin. Die organische Base wird in einem Verhältnis von 0,9 bis 4 Moläquivalenten bezüglich der Verbindung der Formel (I) eingesetzt. Bevorzugt wird die organische Base in einem Verhältnis von 0,7 bis 1,5 Moläquivalenten bezüglich der Verbindung der Formel (I) eingesetzt. Ganz besonders bevorzugt wird die organische Base in einem Verhältnis von 0,9 bis 1,2 Moläquivalenten bezüglich der Verbindung der Formel (I) eingesetzt.

Besonders bevorzugt erfolgt das Auflösen der Verbindung der Formel (I) und die Filtration bei einer Temperatur von 40 bis 120°C. Ganz besonders bevorzugt erfolgt das Auflösen der Verbindung der Formel (I) und die Filtration bei einer Temperatur von 40 bis 80°C.

Die Umsetzung mit Natriumhydroxid oder wässriger Natronlauge oder Natriummethylat oder Natriumethylat oder einem Natrium-Salz in der zweiten Stufe erfolgt bevorzugt in einem Temperaturbereich von 20 bis 120°C, besonders bevorzugt in einem Temperaturbereich von 40 bis 70°C, bei Normaldruck. Die Verbindung der Formel (II) wird aus der erhaltenen Suspension bei einer Temperatur zwischen -20 und 80°C, bevorzugt bei einer Temperatur von 0 bis 20°C, bei Normaldruck durch Filtration isoliert und anschließend getrocknet.

Natriumhydroxid und wässrige Natronlauge und Natriummethylat und Natriumethylat und das Natrium-Salz werden in einem Molverhältnis von 0,8 bis 2 Moläquivalenten bezüglich der Verbindung der Formel (I) eingesetzt. Bevorzugt werden Natriumhydroxid und wässrige Natronlauge und das Natrium-Salz in einem Molverhältnis von 1,0 bis 1,4 Moläquivalenten bezüglich der Verbindung der Formel (I) eingesetzt.

Als Natrium-Salz eigenen sich beispielsweise Salze organischer Säuren wie Natriumcarboxylate, wie beispielsweise Natriumacetat oder Natriumcitrat, oder Salze anorganischer Säuren wie beispielsweise Natriumcarbonat, Natriumhydrogencarbonat, Natriumphosphat, Natriumhydrogenphosphat oder Natriumchlorid. Besonders bevorzugt ist die Verwendung von wässriger Natronlauge oder Natriummethylat oder Natriumethylat. Ganz besonders bevorzugt ist die Verwendung von wässriger Natronlauge.

Weiterer Gegenstand der Erfindung ist ein Verfahren zur Herstellung von 1-[6-(Morpholin-4-yl)pyrimidin-4-yl]-4-(1H-1,2,3-triazol-1-yl)-1H-pyrazol-5-ol (I - Enol-Form) beziehungsweise 2-[6-(Morpholin-4-yl)pyrimidin-4-yl]-4-(1H-1,2,3-triazol-1-yl)-1,2-dihydro-3H-pyrazol-3-on (I - Keto-Form), dadurch gekennzeichnet, dass
a) in der ersten Stufe 1,2,3-Triazol (III) mit Bromessigsäuremethylester (IV-Me-Br) beziehungsweise Bromessigsäureethylester (IV-Et-Br) in Gegenwart von Ethyldiisopropylamin als Base in einem Lösungsmittel in einem Temperaturbereich von 20 bis 80 °C zu Verbindungen der Formeln (V-Me) und (VI-Me) beziehungsweise Verbindungen der Formeln (V-Et) und (VI-Et) umgesetzt wird,
b) in der zweiten Stufe Verbindungen der Formeln (V-Me) und (VI-Me) beziehungsweise Verbindungen der Formeln (V-Et) und (VI-Et), die in einem Verhältnis von mindestens 6:1 zueinander vorliegen, mit Dimethylformamid-Dimethylacetal (XIX-Me) beziehungsweise Dimethylformamid-Diethylacetal (XIX-Et) in einem inerten Lösungsmittel umgesetzt und anschließend durch Abkühlen der Lösung oder durch Abdestillation des Lösungsmittels und Zugabe eines zweiten Lösungsmittels zu Methyl-(2E/Z)-3-(dimethylamino)-2-(1H-1,2,3-triazol-1-yl)acrylat (VII-Me) beziehungsweise Ethyl-(2E/Z)-3-(dimethylamino)-2-(1H-1,2,3-triazol-1-yl)acrylat (VII-Et) kristallisiert werden,
und c)
in der dritten Stufe Methyl-(2E/Z)-3-(dimethylamino)-2-(1H-1,2,3-triazol-1-yl)acrylat (VII-Me) beziehungsweise Ethyl-(2E/Z)-3-(dimethylamino)-2-(1H-1,2,3-triazol-1-yl)acrylat (VII-Et) mit 4-(6-Hydrazinopyrimidin-4-yl)morpholin (XI) in Gegenwart von Trifluoressigsäure in einem inerten Lösungsmittel umgesetzt wird und anschließend die Verbindung der Formel (I) isoliert wird.

Weiterer Gegenstand der Offenbarung ist ein Verfahren zur Herstellung von Methyl-(2E/Z)-3-(dimethylamino)-2-(1H-1,2,3-triazol-1-yl)acrylat (VII-Me) beziehungsweise Ethyl-(2E/Z)-3-(dimethylamino)-2-(1H-1,2,3-triazol-1-yl)acrylat (VII-Et), dadurch gekennzeichnet, dass Verbindungen der Formeln (V-Me) und (VI-Me) beziehungsweise Verbindungen der Formeln (V-Et) und (VI-Et), die in einem Verhältnis von mindestens 6:1 zueinander vorliegen, mit Dimethylformamid-Dimethylacetal (XIX-Me) beziehungsweise Dimethylformamid-Diethylacetal (XIX-Et) in einem inerten Lösungsmittel umgesetzt und anschließend durch Abkühlen der Lösung oder durch Abdestillation des Lösungsmittels und Zugabe eines zweiten Lösungsmittels kristallisiert werden, wobei Verbindungen der Formeln (V-Me) und (VI-Me) beziehungsweise Verbindungen der Formeln (V-Et) und (VI-Et) dadurch hergestellt werden, dass 1,2,3-Triazol (III) mit Bromessigsäuremethylester (IV-Me-Br) beziehungsweise Bromessigsäureethylester (IV-Et-Br) in Gegenwart von Ethyldiisopropylamin als Base in einem Lösungsmittel in einem Temperaturbereich von 20 bis 80 °C umgesetzt wird.

Weiterer Gegenstand der Offenbarung ist Methyl-(2E/Z)-3-(dimethylamino)-2-(1H-1,2,3-triazol-1-yl)acrylat mit der Formel

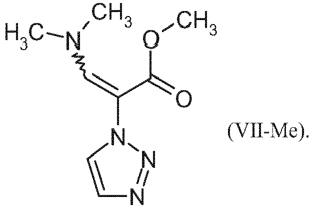

Die einzelnen Stufen des erfindungsgemäßen Verfahrens können bei normalem, erhöhtem oder bei erniedrigtem Druck durchgeführt werden (z.B. von 0,5 bis 5 bar). Sofern nicht anders angegeben arbeitet man im allgemeinen bei Normaldruck.

Die Erfindung wird nachstehend durch ein bevorzugtes Ausführungsbeispiel näher erläutert, auf welches sie jedoch nicht eingeschränkt ist. Soweit nicht anders angegeben, beziehen sich alle Mengenangaben auf Gewichtsprozente.

### Beispiele

### Abkürzungen und Akronyme

- Gew%: Gewichtsprozent
- Fl%: Flächenprozent
- % d. Th.: Prozent der Theorie
- korr.: korrigiert
- unkorr.: unkorrigiert
- min: Minuten
- h: Stunden
- mg: Milligramm
- g: Gramm
- kg: Kilogramm
- l: Liter
- ml: Milliliter
- GC: Gaschromatographie
- HPLC: High Pressure (Performance) Liquid Chromatography; Hochdruckflüssigkeitschromatographie
- s: Singulett
- d: Dublett
- m: Multiplett
- Hz: Hertz

### HPLC-Methode für die Verbindungen der Formel (I) und (II)

Reversed-Phase Methode; Detektion: UV-Bereich
Geräte: Hochleistungsflüssigkeitschromatograph mit thermostatisiertem Säulenofen, UV-Detektor und Datenauswertesystem;
Metallsäule aus Edelstahl: Länge: 15 cm; Innendurchmesser: 3,0 mm; Füllung: z. B. Poroshell 120 EC-C18, 2,7 µm, oder vergleichbar;
Reagenzien: Trifluoressigsäure, für die HPLC; Acetonitril, für die HPLC; 4-Hydroxy-benzoesäure-ethylester, 99 %;
Prüflösung: ca. 25 mg Probe, genau gewogen, mit Wasser/Acetonitril (1:1 v/v) zu 100,0 mL lösen.
Kalibrierlösung: ca. 25 mg Referenzstandard, genau gewogen, mit Wasser/Acetonitril (1:1 v/v) zu 100,0 mL lösen.
Vergleichslösung: Es wird eine Vergleichslösung analog zur Kalibrierlösung hergestellt, die zusätzlich die organischen Verunreinigungen in geringer Menge entsprechend der Spezifikationsgrenze enthält.
HPLC-Bedingungen: Die angegebenen Bedingungen gelten als Richtwerte und sind gegebenenfalls zur Erzielung optimaler Trennungen den technischen Möglichkeiten des Chromatographen und den Eigenschaften der jeweiligen Säule anzupassen.
Elutionsmittel: A. 0,2 %ig Trifluoressigsäure (2,0 mL Trifluoressigsäure mit Wasser zu 1000 mL lösen); B. 0,2 %ig Trifluoressigsäure in Acetonitril (2,0 mL Trifluoressigsäure mit Acetonitril zu 1000 mL lösen); Durchflussrate: 0,5 mL/min;
Temperatur des Säulenofens: 35°C; Detektion: Messwellenlänge 280 nm, Bandbreite: 6 nm; Injektionsvolumen: 3,0 µL; Equilibrierungszeit: 10 min (unter Startbedingungen); Laufzeit des Chromatogramms: 30 min.

| | | | |
|---|---|---|---|
| Gradient | Zeit [min] | % A | % B |
| | 0 | 95 | 5 |
| | 20 | 60 | 40 |
| | 25 | 20 | 80 |
| | 30 | 20 | 80 |

### GC-Methode für die Verbindungen der Formeln (V), (VI), (VII) und (XX)

Gerät/Detektor: Gaschromatograph mit elektronischer Druckkontrolle, Autosampler, FID-Detektor und Datenauswertesystem
Injektortemperatur: 250°C
Liner: Focus-Liner, 4 mm ID, 78,5 x 6,3 mm OD, Fa. SGE, Part.-Nr.: 092219
Säulenfluss: 2 ml/min (constant-flow-modus)
Splitratio / Splitfluss: 20 / 40 ml/min
Säule: HP5 MS UI (Fused Silca, 5% Phenylmethylsiloxan); Länge: 30 m, Innendurchmesser: 0.32 mm, Filmdicke: 1.0 µm
Trägergas: Helium
Temperaturprogramm: Initialtemperatur: 40°C; Initialzeit: 0 min; Rate 10 °C/min, Final 100°C, Haltezeit min; Rate 10 °C/min, Final 300°C, Haltezeit 4 min;
Total (Gesamtlaufzeit): 35 min
Detektortemperatur: 310°C

| | |
|---|---|
| Brenngase: | Oxidizer Flow (synth. Luft): 450 ml/min; Fuel Flow (Wasserstoff): 40 ml/min; Makeup (Stickstoff): 30 ml/min |
| Datenrate: | 10 Hz |

Probenlösungsmittel: Wasser + Acetonitril (2 + 8 V/V)
Prüflösung/ Kalibrierlösung: ca. 5 mg/mL der Substanz mit Lösungsmittel im Ultraschallbad lösen und bis zur Eichmarke auffüllen.
Vergleichslösung: Es werden Vergleichslösungen analog zur Kalibrierlösung hergestellt, die zusätzlich die organischen Verunreinigungen in geringer Menge enthalten.
Injektionsvolumen: 1,0 µl

### GC-Methode für die Verbindungen der Formeln (XI) und (XV)

Gerät/Detektor: Gaschromatograph mit elektronischer Druckkontrolle, Autosampler, FID-Detektor und Datenauswertesystem
Injektortemperatur: 250°C
Liner: Focus-Liner, 4 mm ID, 78,5 x 6,3 mm OD, Fa. SGE, Part.-Nr.: 092219
Säulenfluss: 2 ml/min (constant-flow-modus)
Splitratio / Splitfluss: 20 / 40 ml/min
Säule: HP5 MS UI (Fused Silca, 5% Phenylmethylsiloxan); Länge: 30 m, Innendurchmesser: 0.32 mm, Filmdicke: 1.0 µm
Trägergas: Helium
Temperaturprogramm: Initialtemperatur: 40°C; Initialzeit: 0 min; Rate 10 °C/min, Final 100°C, Haltezeit 5 min; Rate 10 °C/min, Final 300°C, Haltezeit 4 min;
Total (Gesamtlaufzeit): 35 min
Detektortemperatur: 310°C

| | |
|---|---|
| Brenngase: | Oxidizer Flow (synth. Luft): 450 ml/min; Fuel Flow (Wasserstoff): 40 ml/min; Makeup (Stickstoff): 30 ml/min |
| Datenrate: | 10 Hz |

Probenlösungsmittel: Wasser + Acetonitril (1 + 1 V/V)
Prüflösung / Kalibrierlösung: ca. 5 mg/mL der Substanz mit Lösungsmittel im Ultraschallbad lösen und bis zur Eichmarke auffüllen.
Vergleichslösung: Die Vergleichslösungen werden einzeln hergestellt und einzeln injeziert.
Injektionsvolumen: 1,0 µl

### Ionenchromatographie zur Bestimmung von anionischen Verunreinigungen

Gerät: Sympatec Helos; Dispersions Medium: trocken; Druck: 58 psi (4 bar)
Acetat, Bromid, Trifluoracetat; Ionenchromatographie gemäß AM-AAL 61
Geräte: Ionenchromatograph mit Suppressor-System, Leitfähigkeitsdetektor und Chromatographiedatensystem;
Vorsäule: A SUPP 4/5 Guard;
Säule: Trennphase: A SUPP 5; Länge: ca. 250 mm; Innendurchmesser: ca. 4,0 mm;
Reagenzien: Methanol, für die HPLC; Natriumcarbonat, p.a., Natriumhydrogencarbonat, p.a., Milli-Q Wasser, Schwefelsäure, Suprapur;
Prüflösung: Konzentration: 0,1 %; (z. B. 50 mg/50 mL Messkolben); Die Probe wird in 20% des Gesamtvolumens in Methanol gelöst, für drei Minuten im Ultraschallbad behandelt und mit Wasser bis zur Eichmarke aufgefüllt. Anschließend wird über ein ionenfreies Cellulose Acetat Filter (0,45 µm Porengröße) filtriert.
Kalibrierlösungen: Absolute Konzentration: 0,5 mg/L bis 10 mg/L;
IC-Bedingungen: Die angegebenen Bedingungen gelten als Richtwerte und sind gegebenenfalls zur Erzielung optimaler Trennungen den technischen Möglichkeiten des Chromatographen und den Eigenschaften der jeweiligen Säule anzupassen.
Elutionsmittel: 3,2 mmol Natriumcarbonat, 2,4 mmol Natriumhydrogencarbonat/Liter Wasser;
Durchflussrate: 0,7 mL/min;
Detektor: Leitfähigkeitsdetektor;
Bereich: 10 mS/cm;
Fullscale: 50 µS/cm;
Suppressor: Wasser/50 mmol Schwefelsäure;
Injektionsvolumen: 20 µL (Festschleife)
Laufzeit des Chromatogramms: 30 min
Durchführung: Prüflösung und Kalibrierlösungen unter den angegebenen Bedingungen chromatographieren. Die zu bestimmenden und detektierten Peaks im Chromatogramm der Prüflösung müssen in den Retentionszeiten mit den Peaks im Chromatogramm der Kalibrierlösungen übereinstimmen.

| Anion | RT [min] |
|---|---|
| Acetat | ca. 6 |
| Trifluoracetat | ca. 13 |
| Bromid | ca. 14 |

Auswertung: Elektronische Integration der Peakflächen.
Berechnung: Externe Standard Methode (ESTD) mit quadratischer Regression. (Kombinierte Verfahrensschritte, welche unter den Anspruchsumfang fallen, sind erfindungsgemäss).

### Synthese von 4-(6-Hydrazinopyrimidin-4-yl)morpholin (XI)

### Beispiel 1

35.0 kg (234.9 mol) 4,6-Dichlorpyrimidin wurden in 82 kg Wasser bei 20 °C in einem Rührwerksbehälter suspendiert, mit 28.5 kg (281.6 mol) Triethylamin und weiteren 5 kg Wasser versetzt, dann auf 12 °C abgekühlt. Anschließend 14.0 g (279.4 mol) Hydrazinhydrat wurden bei 12 bis 14 °C über etwa 1 h zudosiert. Es wurden weitere 5 kg Wasser zugegeben, 3 h bei 12 °C nachgerührt und dann wurde auf 20 °C erwärmt. Nach 16 h bei 20 °C wurden 23.8 kg (283.3 mol) Natriumhydrogencarbonat, dann 23.6 kg (270.9 mol) Morpholin und 5 kg Wasser zugefügt. Die Mischung wurde mit einer Manteltemperatur von 90 °C erwärmt und 9 h gerührt. Die erhaltene Lösung wurde auf 70 °C abgekühlt und mit 0.14 kg 4-(6-Hydrazinopyrimidin-4-yl)morpholin angeimpft, dann über 5 h auf 3 °C abgekühlt. Die erhaltene Suspension wurde 3 h bei 3 °C gerührt, auf 5 °C abgekühlt und über eine Schälzentrifuge in mehreren Portionen filtriert. Der Produktkuchen wurde jeweils mit kaltem Wasser gewaschen und in einem Mischertrockner im Vakuum bei 40 °C etwa 8 h getrocknet. Ausbeute: 32.9 kg (71.7% d. Th.) der Verbindung der Formel (XI), 98.5 Gew%, 0.5 Fl% Verunreinigungen in Summe, 26 ppm Hydrazin
MS (ESIpos): m/z = 196,0 [M+H]⁺ ;
¹H-NMR (500,13 MHz, d₆-DMSO): 7,95 ppm (bs, 1H), 7,68 (bs, 1H), 5,92 (bs, 1H), 4,14 (bs, 2H), 3,64 (t, 4H), 3,43 (t, 4H).

### Beispiel 2

50.65 g (0.34 mol) 4,6-Dichlorpyrimidin wurden in 140 ml Wasser suspendiert und auf 12 °C abgekühlt. 20.4 g (0.41 mol) Hydrazinhydrat und anschließend 41.3 g (0.41 mol) Triethylamin wurden bei 12 - 15 °C zudosiert. Es wurde bei 12 °C 2 h nachgerührt und über 3 h auf 20 °C erwärmt. Nach 16 h bei 20 °C wurden 34.3 g (0.41 mol) Natriumhydrogencarbonat und dann 34.1 g (0.39 mol) Morpholin zugefügt. Die Mischung wurde auf 80 °C erwärmt, 7 h bei 80 °C und 2 h bei 82 °C gerührt, wobei Leichtsieder abdestilliert wurden. Die erhaltene Lösung wurde über 3 h auf 20 °C abgekühlt. Dabei wurde bei einer Innentemperatur von etwa 70 bis 76 °C mit Produktkristallen angeimpft. Die erhaltene Suspension wurde etwa 12 h bei 20 °C gerührt und anschließend filtriert. Der Produktkuchen wurde zweimal mit je 25 ml Wasser gewaschen und im Vakuum bei 40 °C mindestens 16 h getrocknet. Ausbeute: 43.5 g (65.5% d. Th.) der Verbindung der Formel (XI), 97.8 Gew%

### Beispiel 3

50.65 g (0.34 mol) 4,6-Dichlorpyrimidin wurden in 150 ml Wasser suspendiert und auf 12 °C abgekühlt. 41.3 g (0.41 mol) Triethylamin und anschließend 20.4 g (0.41 mol) Hydrazinhydrat wurden bei 12 bis 15 °C zudosiert. Es wurde bei 12 °C 2 h nachgerührt und über 3 h auf 20 °C erwärmt. Nach ca. 20 h bei 20 °C wurden 34.3 g (0.41 mol) Natriumhydrogencarbonat und dann 34.1 g (0.39 mol) Morpholin zugefügt. Die Mischung wurde auf ca. 78 °C erwärmt und 9 h bei 78 °C gerührt. Die erhaltene Lösung wurde über ca. 5 h auf 20 °C abgekühlt. Die erhaltene Suspension wurde etwa 16 h bei 20 °C gerührt und anschließend filtriert. Der Produktkuchen wurde dreimal mit je 15 ml Wasser gewaschen und im Vakuum bei 40 °C getrocknet. Ausbeute: 51.7 g (78% d. Th.) der Verbindung der Formel (XI), 97 Gew%.

### Beispiel 4

50.65 g (0.34 mol) 4,6-Dichlorpyrimidin wurden in 500 ml Methanol gelöst und auf 0 °C abgekühlt. 41.3 g (0.41 mol) Triethylamin und anschließend 20.4 g (0.41 mol) Hydrazinhydrat wurden bei 0 bis 10 °C zudosiert. Es wurde bei 0 °C 2 h nachgerührt und auf 20 °C erwärmt. Nach 1 h bei 20 °C wurden 41.3 g (0.41 mol) Triethylamin und dann 34.1 g (0.39 mol) Morpholin zugefügt. Die Mischung wurde ca. 70 h unter Rückfluss gerührt. Die erhaltene Lösung wurde auf 20 °C abgekühlt. Dabei wurde bei einer Innentemperatur von etwa 30 °C mit Produktkristallen angeimpft. Die erhaltene Suspension wurde etwa 16 h bei 20 °C gerührt und anschließend filtriert. Der Produktkuchen wurde zweimal mit je 20 ml Methanol gewaschen und im Vakuum bei 40 °C mindestens 16 h getrocknet. Ausbeute: 41 g (62% d. Th. ohne Berücksichtigung des Gehaltes) der Verbindung der Formel (XI), ca. 80 Gew%

### Beispiel 5

50.65 g (0.34 mol) 4,6-Dichlorpyrimidin wurden in 340 ml Isopropanol und 160 ml Wasser vorgelegt. Es wurden 41.3 g (0.41 mol) Triethylamin dazugegeben und auf 10 °C abgekühlt. Anschließend wurden 20.4 g (0.41 mol) Hydrazinhydrat bei 10 bis 15 °C zudosiert. Es wurde 2 h bei 10 bis 15 °C nachgerührt und dann auf 20 °C erwärmt. Nach 3 h bei 20 °C wurden 34.3 g (0.41 mol) Natriumhydrogencarbonat und dann 41.5 g (0.48 mol) Morpholin zugefügt. Die Mischung wurde zum Sieden erhitzt und etwa 400 ml Lösungsmittel werden abdestilliert. Anschließend wurde weitere ca. 6 h bei 84 °C gerührt. Die erhaltene Lösung wurde auf 20 °C abgekühlt. Die erhaltene Suspension wurde bei 20 °C nachgerührt und anschließend filtriert. Der Produktkuchen wurde dreimal mit je 10 ml Wasser gewaschen und im Vakuum bei 40 °C getrocknet. Ausbeute: 40.5 g (61% d. Th.) der Verbindung der Formel (XI), 99 Gew%.

### Synthese von Methyl-1H-1,2,3-triazol-1-ylacetat (V-Me) und Methyl-2H-1,2,3-triazol-1-ylacetat (VI-Me)

### Beispiel 6

Zu einer Lösung von 1.87 kg (14.4 mol) Ethyldiisopropylamin und 500 g (7.2 mol) 1,2,3-Triazol in 5 l Essigsäureethylester wurden 1.34 kg (8.7 mol) Bromessigsäuremethylester über 2.5 bis 3 h so zugetropft, dass die Innentemperatur unterhalb von 43 °C gehalten wurde. Die Reaktionsmischung wurde anschließend 2.5 h bei 40 bis 44 °C, weitere 16 h bei Raumtemperatur und dann 15 Minuten bei 10 °C gerührt. Die erhaltene Suspension wurde filtriert, der Filterkuchen mit 1.5 l Essigsäureethylester gewaschen und die vereinigten Filtrate wurden im Vakuum (bis 30 mbar) bei 45 °C aufkonzentriert. Ausbeute: 1.24 kg orange-rotes Öl (125% d. Th. unkorrigiert), Isomerenverhältnis 7.6 : 1 (Verbindung der Formel (V-Me) : Verbindung der Formel (VI-Me); Gaschromatographie); vollständiger Umsatz bezüglich 1,2,3-Triazol, enthielt noch Ethyldiisopropylamin und Ethyldiisopropylammoniumhydrobromid.

MS (EI+): m/z = 141,0 [M]⁺;

¹H-NMR (500,13 MHz, d₆-DMSO): 8,13 ppm (s, 1H, (V-Me)), 7,77 ppm (s, 1H, (V-Me)), 5,43 ppm (s, 2H, (V-Me)), 3,71 ppm (s, 3H, (V-Me)) sowie Signale mit reduziertem Flächeninhalt für (VI-Me) bei 7,86 ppm (s, 2H, ((VI-Me)), 5,47 ppm (s, 2H, ((VI-Me)), 3,69 ppm (s, 3H, ((VI-Me)).

### Beispiel 7

Zu einer Lösung von 181.5 g (1.4 mol) Ethyldiisopropylamin und 50 g (0.7 mol) 1,2,3-Triazol in 500 ml Essigsäureethylester wurden bei 20 bis 30 °C 130.2 g (0.84 mol) Bromessigsäuremethylester über 30 Minuten zugetropft. Die Reaktionsmischung wurde anschließend 2 h bei 40 °C gerührt. Isomerenverhältnis 8.9 : 1 (Verbindung der Formel (V-Me) : Verbindung der Formel (VI-Me); Gaschromatographie); enthielt noch etwa 2% unumgesetztes 1,2,3-Triazol.

### Beispiel 8

8.5 g (101 mmol) Natriumhydrogencarbonat wurden in 50 ml Aceton suspendiert und mit 5 g (72 mmol) 1,2,3-Triazol versetzt. 9.7 ml (101 mmol) Bromessigsäuremethylester wurden bei ca. 22 °C langsam zudosiert und die Mischung wurde zunächst bei ca. 22 °C etwa 16 h sowie anschließend weitere 3 Tage bei 40 °C gerührt. Die Mischung wurde auf Raumtemperatur abgekühlt und filtriert, der Filterkuchen mit Aceton gewaschen. Die vereinigten Filtrate wurden im Vakuum zu einem gelben Öl eingeengt. Ausbeute: 15.1 g (148% d. Th., unkorrigiert); Isomerenverhältnis 8.4 : 1 (Verbindung der Formel (V-Me) : Verbindung der Formel (VI-Me); Gaschromatographie), enthielt ca. 8% unumgesetztes 1,2,3-Triazol sowie anorganische Salze.

### Beispiel 9

121.6 g (1.45 mol) Natriumhydrogencarbonat wurden in 500 ml Acetonitril suspendiert und mit 51.5 g (0.724 mol) 1,2,3-Triazol versetzt. 97.3 ml (1.01 mol) Bromessigsäuremethylester wurden bei ca. 60 °C über 30 Minuten zudosiert und die Mischung wurde zunächst bei 60 °C etwa 20 h gerührt. Die Mischung wurde auf Raumtemperatur abgekühlt und filtriert, der Filterkuchen mit Acetonitril gewaschen. Die vereinigten Filtrate wurden im Vakuum zu einem gelben Öl eingeengt. Ausbeute: 147.8 g (145% d. Th., unkorrigiert); Isomerenverhältnis 7.5 : 1 (Verbindung der Formel (V-Me) : Verbindung der Formel (VI-Me); Gaschromatographie), enthielt ca. 4% unumgesetztes 1,2,3-Triazol sowie anorganische Salze.

### Synthese von Methyl-(2E/Z)-3-(dimethylamino)-2-(1H-1,2,3-triazol-1-yl)acrylat (VII-Me)

### Beispiel 10

In einem Rührwerksbehälter wurden zu einer Lösung von 74.9 kg (579.5 mol) Ethyldiisopropylamin und 20 kg (289.6 mol) 1,2,3-Triazol in 184.3 kg Essigsäureethylester bei 35 °C 53.6 kg (4.3 mol) Bromessigsäuremethylester über ca. 2 h so zugetropft, dass die Innentemperatur bei 35 bis 37 °C gehalten wurde. Die Reaktionsmischung wurde anschließend 8 h bei 40 °C gerührt. Nach Abkühlen auf 10 °C wurde die erhaltene Suspension filtriert, der Filterkuchen mit 53.3 kg Essigsäureethylester gewaschen und die vereinigten Filtrate wurden im Vakuum bei bis zu 60 °C aufkonzentriert (255 kg Destillat). Der Eindampfrückstand (max. 289.6 mol) wurde bei 40 °C mit 53.1 kg (445.6 mol) N,N-Dimethylformamid-dimethylacetal versetzt und 2 h bei 80 bis 86 °C gerührt. Entstehende Leichtsieder wurden dabei abdestilliert (16 kg Destillat). Anschließend wurde auf 50 °C abgekühlt, 122 kg Aceton zugegeben, auf 50 °C temperieren lassen und heiß von ungelösten Bestandteilen abfiltriert. Der Filterkuchen wurde einmal mit 19 kg Aceton (50 °C) nachgewaschen. Die vereinigten Filtrate wurden durch Destillation aufkonzentriert (97 kg Destillat), mit 47 kg Isopropanol versetzt, erneut durch Destillation bei Normaldruck bis zum Erreichen von etwa 83 °C aufkonzentriert (60 kg Destillat), auf 70 °C abgekühlt und mit 16 kg Isopropanol versetzt. Die Mischung wurde mit 0.15 kg Methyl-(2E/Z)-3-(dimethylamino)-2-(1H-1,2,3-triazol-1-yl)acrylat angeimpft und über 7 h auf 0 °C abgekühlt. Nach einer weiteren Stunde bei 0 °C wurde filtriert, der Filterkuchen zweimal mit einer auf 0 °C abgekühltem Mischung aus 16 kg tert-Butylmethylether und 16 kg Isopropanol gewaschen und bei 40 °C im Vakuum getrocknet. Ausbeute: 43.0 kg (75.7% d. Th. bezogen auf 1,2,3-Triazol) der Verbindung der Formel (VII-Me), 98.2 Gew%, 0.29 Fl% Isomer (GC), 0.2 Gew% Bromid.
MS (ESIpos): m/z = 197,0 [M+H]⁺ ;

¹H-NMR (500,13 MHz, d₆-DMSO): 8,10 ppm (d, 1H), 7,78 (d, 1H), 7,67 (s, 1H), 3,55 (s, 3H), 3,4 - 2,4 (sehr breites s, 3H), 2,4 - 1,7 (sehr breites s, 3H)

### Beispiel 11

Zu einer Lösung von 95.5 g (0.72 mol) Ethyldiisopropylamin und 25 g (0.36 mol) 1,2,3-Triazol in 260 ml Essigsäureethylester wurden 67.1 g (0.43 mol) Bromessigsäuremethylester über ca. 2 h so zugetropft, dass die Innentemperatur bei 35 °C gehalten wurde. Die Reaktionsmischung wurde anschließend 6 h bei 40 °C und weitere 16 h bei 0 °C gerührt. Die erhaltene Suspension wurde filtriert, der Filterkuchen mit 60 ml Essigsäureethylester gewaschen und die vereinigten Filtrate wurden im Vakuum (bis 30 mbar) bei 40 °C aufkonzentriert. Der Eindampfrückstand (59.5 g; 117.1% d. Th. unkorrigiert, max. 0.36 mol) wurde bei 35 °C mit 66 g (1.08 mol) N,N-Dimethylformamid-dimethylacetal versetzt und 2 h bei 83 bis 90 °C gerührt. Entstehende Leichtsieder wurden dabei abdestilliert. Anschließend wurde auf 50 °C abgekühlt, 200 ml Aceton zugegeben, heiß von ungelösten Bestandteilen abfiltriert und zweimal mit je 25 ml Aceton nachgewaschen. Die vereinigten Filtrate wurden durch Destillation aufkonzentriert, mit 75 ml Isopropanol versetzt, erneut durch Destillation bei Normaldruck bis zum Erreichen von etwa 82 °C aufkonzentriert und mit 25 ml Isopropanol versetzt. Bei 70 °C wurde angeimpft und langsam auf Raumtemperatur abgekühlt. Nach Abkühlen auf 0 °C wurde filtriert, der Filterkuchen dreimal mit je 30 ml tert-Butylmethylether/Isopropanol (1:1 v/v) gewaschen und bei 40 °C im Vakuum getrocknet. Ausbeute: 45.8 g (64.4% d. Th. korr.) der Verbindung der Formel (VII-Me), 99.3 Gew%, 0.30 Fl% Isomer (GC), 0.02 Gew% Bromid.

### Beispiel 12

Zu einer Lösung von 124.1 g (0.94 mol) Ethyldiisopropylamin und 50 g (0.72 mol) 1,2,3-Triazol in 520 ml Essigsäureethylester wurden 134.2 g (0.87 mol) Bromessigsäuremethylester über ca. 2 h so zugetropft, dass die Innentemperatur bei 35 °C gehalten wurde. Die Reaktionsmischung wurde anschließend 2 h bei 35 °C, 4 h bei 40 °C und weitere 16 h bei 22 °C gerührt. Nach Abkühlen auf 10 °C wurde die erhaltene Suspension filtriert, der Filterkuchen mit 60 ml Essigsäureethylester gewaschen und die vereinigten Filtrate wurden im Vakuum bei 45 °C aufkonzentriert. Der Eindampfrückstand (123.9 g; 121.9 % d. Th. unkorrigiert, max. 0.72 mol) wurde bei 40 °C mit 133 g (1.08 mol) N,N-Dimethylformamid-dimethylacetal versetzt und 2 h bei 76 bis 88 °C gerührt. Entstehende Leichtsieder wurden dabei abdestilliert. Anschließend wurde auf 50 °C abgekühlt, 400 ml Aceton zugegeben, heiß von ungelösten Bestandteilen abfiltriert und viermal mit je 25 ml Aceton nachgewaschen. Die vereinigten Filtrate wurden durch Destillation aufkonzentriert, mit 150 ml Isopropanol versetzt, erneut durch Destillation bei Normaldruck bis zum Erreichen von etwa 82 °C aufkonzentriert und mit 50 ml Isopropanol versetzt. Bei 70 °C wurde angeimpft und langsam auf Raumtemperatur abgekühlt. Nach Abkühlen auf 0 °C wurde filtriert, der Filterkuchen dreimal mit je 60 ml tert-Butylmethylether/Isopropanol (1:1 v/v) gewaschen und bei 40 °C im Vakuum getrocknet. Ausbeute: 88.8 g (62.8% d. Th. korr.) der Verbindung der Formel (VII-Me), 99.9 Gew%, 0.30 Fl% Isomer (GC), 0.1 Gew% Bromid.

### Beispiel 13

Zu einer Lösung von 1231 ml (7.2 mol) Ethyldiisopropylamin und 250 g (3.6 mol) 1,2,3-Triazol in 2.6 l Essigsäureethylester wurden 671 g (4.3 mol) Bromessigsäuremethylester über ca. 2 h so zugetropft, dass die Innentemperatur bei 35 °C gehalten wurde. Die Reaktionsmischung wurde anschließend 6 h bei 40 °C und weitere 16 h bei 22 °C gerührt. Nach Abkühlen auf 10 °C wurde die erhaltene Suspension filtriert, der Filterkuchen mit 1 l Essigsäureethylester gewaschen und die vereinigten Filtrate wurden im Vakuum bei 45 °C aufkonzentriert. 123.3 g (max. 0.72 mol) des Eindampfrückstandes (616 g; 121 % d. Th. unkorrigiert, max. 3.6 mol) wurde bei 40 °C mit 133 g (1.08 mol) N,N-Dimethylformamid-dimethylacetal versetzt und 2 h bei ca. 80 °C gerührt. Entstehende Leichtsieder wurden dabei abdestilliert. Anschließend wurde auf 50 °C abgekühlt, 400 ml Aceton zugegeben, heiß von ungelösten Bestandteilen abfiltriert und viermal mit je 25 ml Aceton nachgewaschen. Die vereinigten Filtrate wurden durch Destillation aufkonzentriert, mit 150 ml Isopropanol versetzt, erneut durch Destillation bei Normaldruck bis zum Erreichen von etwa 82 °C aufkonzentriert und mit 50 ml Isopropanol versetzt. Bei 70 °C wurde angeimpft und langsam auf Raumtemperatur abgekühlt. Nach Abkühlen auf 0 °C wurde filtriert, der Filterkuchen dreimal mit je 60 ml tert-Butylmethylether/Isopropanol (1:1 v/v) gewaschen und bei 40 °C im Vakuum getrocknet. Ausbeute: 98.4 g (68.4% d. Th. korr.) der Verbindung der Formel (VII-Me), 98.2 Gew%, 0.29 Fl% Isomer (GC), 0.2 Gew% Bromid.

### Beispiel 14

Wie Beispiel 13, allerdings mit nur 115 g (0.94 mol; 1.3 Äquivalente) Dimethylformamid-dimethylacetal. Ausbeute: 91.4 g (64.1% d. Th.) der Verbindung der Formel (VII-Me), 99.1 Gew%, 0.24 Fl% Isomer.

### Beispiel 15

Analog zu Beispiel 13, allerdings mit nur 1.3 Äquivalenten Ethyldiisopropylamin (statt 2 Äquiv.). Ausbeute: 88.8 g (62.8% d. Th.) der Verbindung der Formel (VII-Me), 99.9 Gew%, 0.30 Fl% Isomer

### Beispiel 16

Analog zu Beispiel 13, allerdings wurden nach der Umsetzung mit Dimethylformamid-dimethylacetal 400 ml Tetrahydrofuran (anstelle von Aceton) bei 50 °C zugegeben, heiß von ungelösten Bestandteilen abfiltriert und viermal mit je 25 ml Tetrahydrofuran nachgewaschen. Die vereinigten Filtrate wurden durch Destillation aufkonzentriert (ca. 350 ml Destillat wurden abgenommen), auf RT abgekühlt und 16 h gerührt. Nach Abkühlen auf 0 °C wurde filtriert, der Filterkuchen dreimal mit je ca. 40 ml kaltem Tetrahydrofuran gewaschen und bei 40 °C im Vakuum getrocknet. Ausbeute: 86.8 g (61.4% d. Th.) der Verbindung der Formel (VII-Me), 99.8 Gew%, 0.30 Fl% Isomer.

### Beispiel 17

Analog zu Beispiel 13; nach Zugabe der 400 ml Aceton wurde ebenfalls heiß von ungelösten Bestandteilen abfiltriert und viermal mit je 25 ml Aceton nachgewaschen. Dann allerdings wurden etwa 400 ml Aceton bei Normaldruck abdestilliert und langsam auf 0 °C abgekühlt. Die erhaltene Suspension wurde filtriert, der Filterkuchen 3 mal mit je ca. 40 ml kaltem Aceton gewaschen und im Vakuum bei 40 °C getrocknet. Ausbeute: 81.0 g (57.3% d. Th.) der Verbindung der Formel (VII-Me), 99.8 Gew%, 0.30 Fl% Isomer.

### Beispiel 18

Zu einer Lösung von 93,6 g (0.72 mol) Ethyldiisopropylamin und 25 g (0.36 mol) 1,2,3-Triazol in 260 ml Essigsäureethylester wurden 67.1 g (0.43 mol) Bromessigsäuremethylester über ca. 2 h so zugetropft, dass die Innentemperatur bei 35 °C gehalten wurde. Die Reaktionsmischung wurde anschließend 6 h bei 40 °C, weitere 16 h bei 22 °C gerührt und dann auf 5 °C abgekühlt. Die erhaltene Suspension wurde filtriert, der Filterkuchen mit 100 ml Essigsäureethylester gewaschen und die vereinigten Filtrate wurden bei Normaldruck auf die Hälfte des Volumens durch Destillation aufkonzentriert. Das Konzentrat (198 ml; max. 0.36 mol) wurde bei 45 °C mit 88,9 g (0,72 mol) N,N-Dimethylformamid-dimethylacetal versetzt und 4 h unter Rückfluss (74 bis 82 °C) gerührt. Nach Abkühlen wurde die erhaltene Suspension bei 60 °C filtriert, der Filterrückstand wurde zweimal mit je 25 ml warmen Essigsäureethylester nachgewaschen. Die vereinigten Filtrate wurden durch Destillation bis zu einer Innentemperatur von 80 °C aufkonzentriert, mit 75 ml Isopropanol versetzt, erneut durch Destillation bei Normaldruck bis zum Erreichen von etwa 82 °C aufkonzentriert und mit 25 ml Isopropanol versetzt. Bei 70 °C wurde angeimpft und langsam auf Raumtemperatur abgekühlt. Nach Abkühlen auf 0 °C wurde filtriert, der Filterkuchen dreimal mit je 30 ml kaltem Isopropanol gewaschen und bei 40 °C im Vakuum getrocknet. Ausbeute: 48.9 g (67.5% d. Th. korr.) der Verbindung der Formel (VII-Me), 98.0 Gew%, 99.7 Fl%, 0.20 Fl% Isomer (GC), < 0.1 Gew% Bromid.

### Synthese von Ethyl-(2E/Z)-3-(dimethylamino)-2-(1H-1,2,3-triazol-1-yl)acrylat (VII-Et)

### Beispiel 19

93.6 g (0.72 mol) Ethyldiisopropylamin wurden in 260 ml Essigsäureethylester gelöst und mit 25.0 g (0.36 mol) 1H-1,2,3-Triazol versetzt. Die erhaltene Lösung wurde auf 35 °C erwärmt und 74.8 g (0.43 mol) Bromessigsäureethylester wurden langsam über etwa 90 min zudosiert. Man hielt die Temperatur bei 35 °C über weitere 2 h, rührte dann 4 h bei 40 °C und anschließend 16 h bei ca. 22 °C. Die Suspension wurde auf 10 °C abgekühlt, Ethyldiisopropylamin Hydrobromid abfiltriert und dreimal mit je 40 ml Essigsäureethylester gewaschen. Das Isomerenverhältnis betrug 7.5 : 1 (GC, Verbindung der Formel (V-Et) : Verbindung der Formel (VI-Et). Die vereinigten Filtrate wurden im Vakuum bei etwa 45 °C auf etwa 71 g eines beweglichen Öls aufkonzentriert, mit 79.9 g (0.54 mol) N,N-Dimethylformamid-diethylacetal versetzt und bei 88 bis 95 °C 2 h gerührt, wobei entstehende Leichtsieder abdestilliert wurden. Nach Abkühlen auf ca. 50 °C wurden 200 ml Aceton zugegeben, ca. 30 min bei 50 °C nachgerührt und von Ungelöstem heiß abfiltriert. Der zurückbleibende Feststoff wurde viermal mit je 12.5 ml heißem Aceton gewaschen. Die vereinigten Filtrate wurden durch Destillation aufkonzentriert, mit 75 ml Isopropanol versetzt und erneut zum Sieden erhitzt. Bis zum Erreichen einer Innentemperatur von etwa 83 °C wurden Leichtsieder weiter abdestilliert. Es wurden 25 ml Isopropanol zugegeben und langsam auf 20 °C, später auf 0 °C abgekühlt. Es wurde keine Kristallisation beobachtet. Nach Einengen im Vakuum wurde in 125 ml Methyl-tert-butylether/Isopropanol (3:1 v/v) heiß gelöst und erneut langsam auf 20 °C abgekühlt. Es bildeten sich eine geringe Menge Kristallisat (1.5 g nach Filtration und Trocknung, kein Produkt). Die Mutterlauge wurde erneut aufkonzentriert und durch Behandlung mit 200 ml Methyl-tert-butylether und 50 ml Isopropanol konnte eine Kristallsuspension erhalten werden. Das Produkt wurde durch Filtration isoliert, mit dreimal mit je 100 ml Methyl-tert-butylether/Isopropanol (9:1 v/v) gewaschen und im Vakuum bei 40 °C getrocknet. Ausbeute: 29.6 g Verbindung der Formel (VII-Et) (39% d. Th.), 98.8 F1%, 0.7 % Isomer Verbindung der Formel (XX-Et).

### Synthese von 1-[6-(Morpholin-4-vl)pyrimidin-4-yl]-4-(1H-1,2,3-triazol-1-yl)-1H-pyrazol-5-ol (Enol-Form) bzw. 2-[6-(Morpholin-4-yl)pyrimidin-4-yl]-4-(1H-1,2,3-triazol-1-yl)-1,2-dihydro-3H-pyrazol-3-on (Keto-Form) (I)

### Beispiel 20

In einem Rührwerkskessel wurden 42.0 kg (215.1 mol) 4-(6-Hydrazinopyrimidin-4-yl)morpholin und 44.0 kg (224.2 mol) Methyl-(2E/Z)-3-(dimethylamino)-2-(1H-1,2,3-triazol-1-yl)acrylat in 378 kg Essigsäureethylester suspendiert, mit 12.1 kg (106.1 mol) Trifluoressigsäure versetzt und 26 h bei einer Manteltemperatur von 90 °C unter Rückfluss (78 bis 81 °C) erhitzt. Die erhaltene Suspension wurde auf 0 °C abgekühlt, 1 h bei 0 °C gerührt und filtriert. Der Filterkuchen wurde mit 53 kg Essigsäureethylester gewaschen und im Vakuum bei bis zu 45 °C getrocknet. Der Filterkuchen wurde mit einer Mischung von 355 kg Wasser und 11.7 kg Essigsäure versetzt, suspendiert und bei 50 bis 54 °C für 1 h gerührt. Nach Abkühlen auf 24 °C wurde die Suspension filtriert. Der Filterkuchen wurde zunächst mit 90 kg Wasser, dann zweimal mit je 50 kg Methanol gewaschen und anschließend im Vakuum bei 35 bis 45 °C getrocknet. Ausbeute: 57.4 kg (84.9% d. Th.) der Verbindung der Formel (I), 99.9 Fl%.

MS (ESI+): m/z = 315,0 [M+H]⁺;

¹H-NMR (500,13 MHz, d₆-DMSO): 8,55 ppm (s, 1H), 8,38 ppm (d, 1H, 0,6 Hz), 8,27 ppm (s, 1H), 7,86 ppm (d, 1H, 0,6 Hz), 7,42 ppm (s, 1H), 3,71 ppm (s, 8H)

### Beispiel 21

10.0 g (51.2 mmol) 4-(6-Hydrazinopyrimidin-4-yl)morpholin und 10.15 g (51.2 mmol) Methyl-3-(dimethylamino)-2-(1H-1,2,3-triazol-1-yl)acrylat wurden in 100 ml Essigsäureethylester suspendiert, mit 2.92 g (25.6 mmol) Trifluoressigsäure versetzt und 24 h unter Rückfluss erhitzt. Die erhaltene Suspension wurde auf 0 °C abgekühlt und filtriert. Der Filterkuchen wurde zweimal mit je 4 ml Essigsäureethylester gewaschen und gut trocken gesaugt. Der Filterkuchen wurde in 100 ml Wasser suspendiert, mit 2.4 ml Essigsäure angesäuert und bei 50 °C für ca. 30 min gerührt. Nach Abkühlen auf 20 °C wurde die Suspension filtriert, der Filterkuchen zweimal mit je 10 ml Wasser gewaschen und im Vakuum bei 40 °C getrocknet. Ausbeute: 13.5 g (83.9% d. Th.) der Verbindung der Formel (I), 99.9 Fl%, 100 Gew%.

### Beispiel 22

10.0 g (51.2 mmol) 4-(6-Hydrazinopyrimidin-4-yl)morpholin und 12.2 g (61.5 mmol) Methyl-3-(dimethylamino)-2-(1H-1,2,3-triazol-1-yl)acrylat wurden in 100 ml Essigsäureethylester suspendiert, mit 2.92 g (25.6 mmol) Trifluoressigsäure versetzt und 24 h unter Rückfluss erhitzt. Die erhaltene Suspension wurde auf 0 °C abgekühlt und filtriert. Der Filterkuchen wurde zweimal mit je 4 ml Essigsäureethylester gewaschen und gut trocken gesaugt. Der Filterkuchen wurde in 100 ml Wasser suspendiert, mit 2.4 ml Essigsäure angesäuert und bei 50 °C für ca. 30 min gerührt. Nach Abkühlen auf 20 °C wurde die Suspension filtriert, der Filterkuchen zweimal mit je 10 ml Wasser gewaschen und im Vakuum bei 40 °C getrocknet. Ausbeute: 14.4 g (85.8% d. Th.) der Verbindung der Formel (I), 99.8 Fl%, 95.9 Gew%.

### Beispiel 23

10.0 g (51.2 mmol) 4-(6-Hydrazinopyrimidin-4-yl)morpholin und 10.7 g (53.8 mmol) Methyl-3-(dimethylamino)-2-(1H-1,2,3-triazol-1-yl)acrylat wurden in 100 ml n-Butylacetat suspendiert, mit 2.92 g (25.6 mmol) Trifluoressigsäure versetzt und 6 h bei Rückfluss (ca. 120 °C) gerührt. Die erhaltene Suspension wurde 16 h bei 20 °C und dann 1 h bei 0 °C gerührt und filtriert. Der Filterkuchen wurde zweimal mit je 4 ml kaltem Essigsäureethylester gewaschen und bei 40 °C im Vakuum getrocknet. Der getrocknete Filterkuchen (15.6 g) wurde in 100 ml Wasser suspendiert, mit 2.4 ml Essigsäure versetzt und 30 min bei 50 °C gerührt. Nach Abkühlen auf 20 °C wurde die Suspension filtriert, der Filterkuchen mit zweimal mit je 10 ml Wasser gewaschen und im Vakuum bei 40 °C getrocknet. Ausbeute: 13.9 g (86.3% d. Th.) der Verbindung der Formel (I), 99.7 Fl%, 99.0 Gew%.

### Beispiel 24

10.0 g (51.2 mmol) 4-(6-Hydrazinopyrimidin-4-yl)morpholin und 10.66 g (53.8 mmol) Methyl-3-(dimethylamino)-2-(1H-1,2,3-triazol-1-yl)acrylat wurden in 100 ml n-Butanol suspendiert, mit 2.92 g (25.6 mmol) Trifluoressigsäure versetzt und 8 h bei ca. 117 °C gerührt. Die erhaltene Suspension wurde auf 0 °C abgekühlt und filtriert. Der Filterkuchen wurde zweimal mit je 4 ml n-Butanol gewaschen, im Vakuum bei 40 °C getrocknet, anschließend in 100 ml Wasser suspendiert, mit 2.4 ml Essigsäure angesäuert und bei 50 °C für ca. 30 min gerührt. Nach Abkühlen auf 20 °C wurde die Suspension filtriert, der Filterkuchen zweimal mit je 10 ml Wasser gewaschen und im Vakuum bei 40 °C getrocknet. Ausbeute: 13.2 g (81.7% d. Th.) der Verbindung der Formel (I), 99.7 F1%, 99.0 Gew%.

### Beispiel 25

10.0 g (51.2 mmol) 4-(6-Hydrazinopyrimidin-4-yl)morpholin und 10.7 g (53.8 mmol) Methyl-3-(dimethylamino)-2-(1H-1,2,3-triazol-1-yl)acrylat wurden in 100 ml Essigsäureethylester suspendiert, mit 7.01 g (61.5 mmol) Trifluoressigsäure versetzt und 2 h unter Rückfluss erhitzt. Anschließend wurden 11.4 g (112.7 mmol) Triethylamin zugegeben und für weitere 4 h bei Rückfluss gerührt. Die erhaltene Suspension wurde auf 0 °C abgekühlt, 1 h bei 0 °C gerührt und filtriert. Der Filterkuchen wurde zweimal mit je 4 ml Essigsäureethylester gewaschen und gut trocken gesaugt. Der Filterkuchen wurde in 100 ml Wasser suspendiert, mit 3.5 ml Essigsäure auf pH 5 angesäuert und bei 50 °C für ca. 30 min gerührt. Nach Abkühlen auf 20 °C wurde die Suspension filtriert, der Filterkuchen zweimal mit je 10 ml Wasser gewaschen und im Vakuum bei 40 °C getrocknet. Ausbeute: 13.5 g (83.9% d. Th.) der Verbindung der Formel (I), 99.8 Fl%.

### Synthese von Natrium-1-[6-(morpholin-4-yl)pyrimidin-4-yl]-4-(1H-1,2,3-triazol-1-yl)-1H-pyrazol-5-olat (II)

### Beispiel 26

In einem Rührwerkskessel wurden 55 kg (175,0 mol) der Verbindung der Formel (I) in einer Mischung aus 200 kg Methanol und 30 kg Wasser suspendiert, mit 17,8 kg (175,9 mmol) Triethylamin versetzt, auf 60 °C erwärmt, etwa 1 h nachgerührt und zur Abtrennung ungelöster Bestandteile heiß filtriert. Der Filterkuchen wurde mit 15 kg Methanol (60 °C) gewaschen. Zu den vereinigten Filtraten wurden bei 60 °C 18,7 kg (210,4 mmol) 45%ige Natronlauge langsam zudosiert und 5 kg Methanol zugegeben. Es wurden 0,12 kg Natrium-1-[6-(morpholin-4-yl)pyrimidin-4-yl]-4-(1H-1,2,3-triazol-1-yl)-1H-pyrazol-5-olat als Impfkristalle zugegeben, die Mischung wurde bei 60 °C 1 h nachgerührt und über etwa 2 h auf 24 °C abgekühlt. Bei dieser Temperatur wurde die Mischung 8 h gerührt, anschließend über etwa 1 h auf 0 °C abgekühlt und portionsweise über eine Zentrifuge filtriert. Die Filterkuchen wurde insgesamt mit einer Mischung aus 24 kg Wasser und 168kg Methanol sowie jeweils etwa 23 kg Methanol gewaschen und gemeinsam in einem Kugeltrockner bei 40 °C im Vakuum 8 h getrocknet. Ausbeute: 57,6 kg (97,9% d. Th.) der Verbindung der Formel (II); 100 Gew%, 99,9 Fl%.

¹H-NMR (500,13 MHz, d₆-DMSO): 8,98 ppm (d, 1H, 1,4 Hz), 8,72 ppm (s, 1H), 8,68 ppm (s, 1H), 8,64 ppm (d, 1H, 1,4 Hz), 7,77 ppm (s, 1H), 4,00 - 4,25 ppm (m, 8H).

### Beispiel 27

12.4 g (39.5 mmol) der Verbindung der Formel (I) wurden in einer Mischung aus 63 ml Methanol und 7 ml Wasser suspendiert, mit 4.0 g (39.6 mmol) Triethylamin versetzt und auf 60 °C erwärmt. 4.2 g (47.4 mmol) 45%ige Natronlauge wurden bei 60 °C langsam zudosiert. Nach Zugabe von Impfkristallen wurde die Mischung auf 50 °C abgekühlt, bei dieser Temperatur 1 h gerührt und anschließend langsam auf ca. 5 °C abgekühlt. Die erhaltene Suspension wurde filtriert, der Filterkuchen zweimal mit je etwa 4 ml Methanol/Wasser (9:1 v/v) gewaschen und bei 40 °C im Vakuum 16 h getrocknet. Ausbeute: 13.1 g (98.7% d. Th.) der Verbindung der Formel (II); 100 Gew%, 99.9 Fl%.

### Beispiel 28

12.4 g (39.5 mmol) der Verbindung der Formel (I) wurden in einer Mischung aus 63 ml Methanol und 7 ml Wasser suspendiert, mit 4.0 g (39.6 mmol) Triethylamin versetzt und auf 60 °C erwärmt. Die so erhaltene Lösung wurde zu einer Lösung aus 1.9 g (47.4 mmol) Natriumhydroxid in 50 ml Methanol bei 60 °C über etwa 30 min dosiert. Nach Zugabe von Impfkristallen wurde die Mischung auf 50 °C abgekühlt, bei dieser Temperatur 1 h gerührt und anschließend langsam auf ca. °C abgekühlt. Die erhaltene Suspension wurde filtriert, der Filterkuchen zweimal mit je etwa 10 ml Methanol/Wasser (9:1 v/v) gewaschen und bei 40 °C im Vakuum 18 h getrocknet. Ausbeute: 12.7 g (95.4% d. Th.) der Verbindung der Formel (II); 100 Gew%, 99.9 F1%.

### Beispiel 29

12.4 g (39.5 mmol) der Verbindung der Formel (I) wurden in einer Mischung aus 63 ml Methanol und 7 ml Wasser suspendiert, mit 4.0 g (39.6 mmol) Triethylamin versetzt und auf 60 °C erwärmt. Die so erhaltene Lösung wurde zu 8.5 g (47.4 mmol) methanolischer Natriummethylat-Lösung (30 %ig) in 50 ml Methanol bei 60 °C über etwa 30 min dosiert. Nach Zugabe von Impfkristallen wurde die Mischung auf 50 °C abgekühlt, bei dieser Temperatur 1 h gerührt und anschließend langsam auf ca. 5 °C abgekühlt. Die erhaltene Suspension wurde filtriert, der Filterkuchen zweimal mit je etwa 4 ml Methanol/Wasser (9:1 v/v) gewaschen und bei 40 °C im Vakuum 16 h getrocknet. Ausbeute: 12.8 g (96.5% d. Th.) der Verbindung der Formel (II); 100 Gew%, 99.9 Fl%.

## Patentansprüche

1. Verfahren zur Herstellung von 1-[6-(Morpholin-4-yl)pyrimidin-4-yl]-4-(1H-1,2,3-triazol-1-yl)-1H-pyrazol-5-ol (I - Enol-Form) beziehungsweise 2-[6-(Morpholin-4-yl)pyrimidin-4-yl]-4-(1H-1,2,3-triazol-1-yl)-1,2-dihydro-3H-pyrazol-3-on (I- Keto-Form), **dadurch gekennzeichnet, dass**
a) in der ersten Stufe 1,2,3-Triazol (III) mit Bromessigsäuremethylester (IV-Me-Br) beziehungsweise Bromessigsäureethylester (IV-Et-Br) in Gegenwart von Ethyldiisopropylamin als Base in einem Lösungsmittel in einem Temperaturbereich von 20 bis 80 °C zu den Verbindungen Methyl-1H-1,2,3-triazol-1-ylacetat (V-Me) und Methyl-2H-1,2,3-triazol-1-ylacetat (VI-Me) beziehungsweise den Verbindungen Ethyl-1H-1,2,3-triazol-1-yl-acetat (V-Et) und Ethyl-2H-1,2,3-triazol-1-ylacetat (VI-Et) umgesetzt wird,
b) in der zweiten Stufe die Verbindungen Methyl-1H-1,2,3-triazol-1-ylacetat (V-Me) und Methyl-2H-1,2,3-triazol-1-ylacetat (VI-Me) beziehungsweise die Verbindungen Ethyl-1H-1,2,3-triazol-1-yl-acetat (V-Et) und Ethyl-2H-1,2,3-triazol-1-ylacetat (VI-Et), die in einem Verhältnis von mindestens 6:1 zueinander vorliegen, mit Dimethylformamid-Dimethylacetal (XIX-Me) beziehungsweise Dimethylformamid-Diethylacetal (XIX-Et) in einem inerten Lösungsmittel umgesetzt und anschließend durch Abkühlen der Lösung oder durch Abdestillation des Lösungsmittels und Zugabe eines zweiten Lösungsmittels zu Methyl-(2E/Z)-3-(dimethylamino)-2-(1H-1,2,3-triazol-1-yl)acrylat (VII-Me) beziehungsweise Ethyl-(2E/Z)-3-(dimethylamino)-2-(1H-1,2,3-triazol-1-yl)acrylat (VII-Et) kristallisiert werden,
und
c) in der dritten Stufe Methyl-(2E/Z)-3-(dimethylamino)-2-(1H-1,2,3-triazol-1-yl)acrylat (VII-Me) beziehungsweise Ethyl-(2E/Z)-3-(dimethylamino)-2-(1H-1,2,3-triazol-1-yl)acrylat (VII-Et) mit 4-(6-Hydrazinopyrimidin-4-yl)morpholin (XI) in Gegenwart von Trifluoressigsäure in einem inerten Lösungsmittel umgesetzt wird und anschließend die Verbindung der Formel (I) isoliert wird.

2. Verfahren nach Anspruch 1, **dadurch gekennzeichnet, dass** zur Herstellung von 1-[6-(Morpholin-4-yl)pyrimidin-4-yl]-4-(1H-1,2,3-triazol-1-yl)-1H-pyrazol-5-ol (I - Enol-Form) beziehungsweise 2-[6-(Morpholin-4-yl)pyrimidin-4-yl]-4-(1H-1,2,3-triazol-1-yl)-1,2-dihydro-3H-pyrazol-3-on (I - Keto-Form) Methyl-(2E/Z)-3-(dimethylamino)-2-(1H-1,2,3-triazol-1-yl)acrylat (VII-Me) oder Ethyl-(2E/Z)-3-(dimethylamino)-2-(1H-1,2,3-triazol-1-yl)acrylat (VII-Et) mit 4-(6-Hydrazinopyrimidin-4-yl)morpholin (XI) in Gegenwart von Trifluoressigsäure in einem inerten Lösungsmittel und anschließender Zugabe von Triethylamin umgesetzt wird und anschließend die Verbindung der Formel (I) isoliert wird.

3. Verfahren nach Anspruch 2, **dadurch gekennzeichnet, dass** das Reaktionsgemisch nach Zugabe von Triethylamin bei einer Temperatur von 20 bis 90°C gehalten wird.

4. Verfahren nach einem der Ansprüche 1 bis 3, **dadurch gekennzeichnet, dass** die Aufreinigung des erhaltenen Rohproduktes der Verbindung der Formel (I) mit Wasser und einer Säure bei einem pH-Wert von 4 bis 5,5 erfolgt.

5. Verfahren nach einem der Ansprüche 1 bis 4, **dadurch gekennzeichnet, dass** die so erhaltene Verbindung der Formel (I) in einem anschließenden Schritt mit Natriumhydroxid oder wässriger Natronlauge oder Natriummethylat oder Natriumethylat oder einem Natrium-Salz zu Natrium-1-[6-(morpholin-4-yl)pyrimidin-4-yl]-4-(1H-1,2,3-triazol-1-yl)-1H-pyrazol-5-olat (II) umgesetzt wird.

6. Verfahren nach einem der Ansprüche 1 bis 4 wobei 4-(6-Hydrazinopyrimidin-4-yl)morpholin (XI) dadurch hergestellt wird, dass in der ersten Stufe zunächst 4,6-Dichlorpyrimidin (VIII) mit Hydrazinhydrat (XII-Hydrat) in einem Lösungsmittel, gegebenenfalls in Gegenwart einer Hilfsbase, umgesetzt und das erhaltene Reaktionsgemisch ohne Isolierung der gebildeten 4-Chlor-6-hydrazinopyrimidins (XV) in einer zweiten Stufe nach Zugabe von Morpholin (IX) und einer weiteren Hilfsbase erhitzt, sowie die Verbindung der Formel (XI) anschließend nach Kristallisation isoliert wird.

7. Verfahren nach Anspruch 6, **dadurch gekennzeichnet, dass** die Umsetzung mit Triethylamin oder Ethyldiisopropylamin als Hilfsbase durchgeführt wird.

8. Verfahren nach einem der Ansprüche 6 oder 7, **dadurch gekennzeichnet, dass** die Umsetzung mit Natriumhydrogencarbonat, Kaliumhydrogencarbonat, Natriumhydroxid oder Kaliumhydroxid als weiterer Hilfsbase durchgeführt wird.

9. Verfahren nach einem der Ansprüche 1 bis 4 wobei Methyl-(2E/Z)-3-(dimethylamino)-2-(1H-1,2,3-triazol-1-yl)acrylat (VII-Me) beziehungsweise Ethyl-(2E/Z)-3-(dimethylamino)-2-(1H-1,2,3-triazol-1-yl)acrylat (VII-Et) dadurch hergestellt wird, dass die Verbindungen Methyl-1H-1,2,3-triazol-1-ylacetat (V-Me) und Methyl-2H-1,2,3-triazol-1-ylacetat (VI-Me) beziehungsweise die Verbindungen Ethyl-1H-1,2,3-triazol-1-yl-acetat (V-Et) und Ethyl-2H-1,2,3-triazol-1-ylacetat (VI-Et), die in einem Verhältnis von mindestens 6:1 zueinander vorliegen, mit Dimethylformamid-Dimethylacetal (XIX-Me) beziehungsweise Dimethylformamid-Diethylacetal (XIX-Et) in einem inerten Lösungsmittel umgesetzt und anschließend durch Abkühlen der Lösung oder durch Abdestillation des Lösungsmittels und Zugabe eines zweiten Lösungsmittels kristallisiert werden.

10. Verfahren nach Anspruch 9 wobei die Verbindungen Methyl-1H-1,2,3-triazol-1-ylacetat (V-Me) und Methyl-2H-1,2,3-triazol-1-ylacetat (VI-Me) beziehungsweise die Verbindungen Ethyl-1H-1,2,3-triazol-1-yl-acetat (V-Et) und Ethyl-2H-1,2,3-triazol-1-ylacetat (VI-Et) dadurch hergestellt werden, dass 1,2,3-Triazol (III) mit Bromessigsäuremethylester (IV-Me-Br) beziehungsweise Bromessigsäureethylester (IV-Et-Br) in Gegenwart von Ethyldiisopropylamin als Base in einem Lösungsmittel in einem Temperaturbereich von 20 bis 80 °C umgesetzt wird.

## Claims

1. Process for preparing 1-[6-(morpholin-4-yl)pyrimidin-4-yl]-4-(1H-1,2,3-triazol-1-yl)-1H-pyrazol-5-ol (I - enol form) or 2-[6-(morpholin-4-yl)pyrimidin-4-yl]-4-(1H-1,2,3-triazol-1-yl)-1,2-dihydro-3H-pyrazol-3-one (I - keto form), **characterized in that**
a) in the first step, 1,2,3-triazole (III) is reacted with methyl bromoacetate (IV-Me-Br) or ethyl bromoacetate (IV-Et-Br) in the presence of ethyldiisopropylamine as base in a solvent in the temperature range from 20 to 80°C to form the compounds methyl-1H-1,2,3-triazol-1-yl acetate (V-Me) and methyl-2H-1,2,3-triazol-1-yl acetate (VI-Me) or the compounds ethyl-1H-1,2,3-traizol-1-yl acetate (V-Et) and ethyl-2H-1,2,3-triazol-1-yl acetate (VI-Et),
b) in the second step, the compounds methyl-1H-1,2,3-triazol-1-yl acetate (V-Me) and methyl-2H-1,2,3-triazol-1-yl acetate (VI-Me) or the compounds ethyl-1H-1,2,3-traizol-1-yl acetate (V-Et) and ethyl-2H-1,2,3-triazol-1-yl acetate (VI-Et), which are present in a ratio of at least 6:1 to one another, are reacted with dimethylformamide dimethyl acetal (XIX-Me) or dimethylformamide diethyl acetal (XIX-Et) in an inert solvent and are subsequently crystallized by cooling the solution or by distilling off the solvent and adding a second solvent to form methyl (2E/Z)-3-(dimethylamino)-2-(1H-1,2,3-triazol-1-yl)acrylate (VII-Me) or ethyl (2E/Z)-3-(dimethylamino)-2-(1H-1,2,3-triazol-1-yl)acrylate (VII-Et),
and
c) in the third step, methyl (2E/Z)-3-(dimethylamino)-2-(1H-1,2,3-triazol-1-yl)acrylate (VII-Me) or ethyl (2E/Z)-3-(dimethylamino)-2-(1H-1,2,3-triazol-1-yl)acrylate (VII-Et) is reacted with 4-(6-hydrazinopyrimidin-4-yl)morpholine (XI) in the presence of trifluoroacetic acid in an inert solvent and the compound of the formula (I) is subsequently isolated.

2. Process according to Claim 1, **characterized in that**, for preparing 1-[6-(morpholin-4-yl)pyrimidin-4-yl]-4-(1H-1,2,3-triazol-1-yl)-1H-pyrazol-5-ol (I - enol form) or 2-[6-(morpholin-4-yl)pyrimidin-4-yl]-4-(1H-1,2,3-triazol-1-yl)-1,2-dihydro-3H-pyrazol-3-one (I - keto form), methyl (2E/Z)-3-(dimethylamino)-2-(1H-1,2,3-triazol-1-yl)acrylate (VII-Me) or ethyl (2E/Z)-3-(dimethylamino)-2-(1H-1,2,3-triazol-1-yl)acrylate (VII-Et) is reacted with 4-(6-hydrazinopyrimidin-4-yl)morpholine (XI) in the presence of trifluoroacetic acid in an inert solvent with subsequent addition of triethylamine and the compound of the formula (I) is subsequently isolated.

3. Process according to Claim 2, **characterized in that** the reaction mixture is maintained at a temperature of from 20 to 90°C after addition of triethylamine.

4. Process according to any of Claims 1 to 3, **characterized in that** the purification of the resulting crude produce of the compound of the formula (I) is carried out using water and an acid at a pH of from 4 to 5.5.

5. Process according to any of Claims 1 to 4, **characterized in that** the compound of the formula (I) obtained in this way is, in a subsequent step, reacted with sodium hydroxide or aqueous sodium hydroxide solution or sodium methoxide or sodium ethoxide or a sodium salt to form sodium 1-[6-(morpholin-4-yl)pyrimidin-4-yl]-4-(1H-1,2,3-triazol-1-yl)-1H-pyrazol-5-olate (II).

6. Process according to any of Claims 1 to 4, wherein 4-(6-hydrazinopyrimidin-4-yl)morpholine (XI) is prepared by, in the first stage, firstly reacting 4,6-dichloropyrimidine (VIII) with hydrazine hydrate (XII hydrate) in a solvent, optionally in the presence of an auxiliary base, and heating the resulting reaction mixture without isolation of the resulting 4-chloro-6-hydrazinopyrimidine (XV) in a second stage after addition of morpholine (IX) and a further auxiliary base and subsequently isolating the compound of the formula (XI) after crystallization.

7. Process according to Claim 6, **characterized in that** the reaction is carried out using triethylamine or ethyldiisopropylamine as auxiliary base.

8. Process according to either of Claims 6 and 7, **characterized in that** the reaction is carried out using sodium hydrogencarbonate, potassium hydrogencarbonate, sodium hydroxide or potassium hydroxide as further auxiliary base.

9. Process according to any of Claims 1 to 4, wherein methyl (2E/Z)-3-(dimethylamino)-2-(1H-1,2,3-triazol-1-yl)acrylate (VII-Me) or ethyl (2E/Z)-3-(dimethylamino)-2-(1H-1,2,3-triazol-1-yl)acrylate (VII-Et) is prepared by reacting the compounds methyl-1H-1,2,3-triazol-1-yl acetate (V-Me) and methyl-2H-1,2,3-triazol-1-yl acetate (VI-Me) or the compounds ethyl-1H-1,2,3-traizol-1-yl acetate (V-Et) and ethyl-2H-1,2,3-triazol-1-yl acetate (VI-Et), which are present in a ratio of at least 6:1 to one another, with dimethylformamide dimethyl acetal (XIX-Me) or dimethylformamide diethyl acetal (XIX-Et) in an inert solvent and subsequently crystallizing the product by cooling the solution or by distilling off the solvent and adding a second solvent.

10. Process according to Claim 9, wherein the compounds methyl-1H-1,2,3-triazol-1-yl acetate (V-Me) and methyl-2H-1,2,3-triazol-1-yl acetate (VI-Me) or the compounds ethyl-1H-1,2,3-traizol-1-yl acetate (V-Et) and ethyl-2H-1,2,3-triazol-1-yl acetate (VI-Et) are prepared by reacting 1,2,3-triazole (III) with methyl bromoacetate (IV-Me-Br) or ethyl bromoacetate (IV-Et-Br) in the presence of ethyldiisopropylamine as base in a solvent in the temperature range from 20 to 80°C.

## Revendications

1. Procédé de fabrication de 1-[6-(morpholin-4-yl)pyrimidin-4-yl]-4-(1H-1,2,3-triazol-1-yl)-1H-pyrazol-5-ol (I - forme énol) ou de 2-[6-(morpholin-4-yl)pyrimidin-4-yl]-4-(1H-1,2,3-triazol-1-yl)-1,2-dihydro-3H-pyrazol-3-one (I - forme céto), **caractérisé en ce que**
a) lors de la première étape, du 1,2,3-triazole (III) est mis en réaction avec de l'ester méthylique de l'acide bromoacétique (IV-Me-Br) ou de l'ester éthylique de l'acide bromoacétique (IV-Et-Br) en présence d'éthyldiisopropylamine en tant que base dans un solvant dans une plage de température de 20 à 80 °C pour former les composés acétate de méthyl-1H-1,2,3-triazol-1-yle (V-Me) et acétate de méthyl-2H-1,2,3-triazol-1-yle (VI-Me) ou les composés acétate d'éthyl-1H-1,2,3-triazol-1-yle (V-Et) et acétate d'éthyl-2H-1,2,3-triazol-1-yle (VI-Et),
b) lors de la deuxième étape, les composés acétate de méthyl-1H-1,2,3-triazol-1-yle (V-Me) et acétate de méthyl-2H-1,2,3-triazol-1-yle (VI-Me) ou les composés acétate d'éthyl-1H-1,2,3-triazol-1-yle (V-Et) et acétate d'éthyl-2H-1,2,3-triazol-1-yle (VI-Et), qui sont présents en un rapport d'au moins 6:1 les uns par rapport aux autres, sont mis en réaction avec du diméthylformamide-diméthylacétal (XIX-Me) ou du diméthylformamide-diéthylacétal (XIX-Et) dans un solvant inerte, puis cristallisés par refroidissement de la solution ou par élimination par distillation du solvant et ajout d'un deuxième solvant en acrylate de méthyl-(2E/2)-3-(diméthylamino)-2-(1H-1,2,3-triazol-1-yle) (VII-Me) ou acrylate d'éthyl-(2E/Z)-3-(diméthylamino)-2-(1H-1,2,3-triazol-1-yle) (VII-Et),
et
c) lors de la troisième étape, l'acrylate de méthyl-(2E/2)-3-(diméthylamino)-2-(1H-1,2,3-triazol-1-yle) (VII-Me) ou l'acrylate d'éthyl-(2E/Z)-3-(diméthylamino)-2-(1H-1,2,3-triazol-1-yle) (VII-Et) est mis en réaction avec de la 4-(6-hydrazinopyrimidin-4-yl)morpholine (XI) en présence d'acide trifluoroacétique dans un solvant inerte, puis le composé de formule (I) est isolé.

2. Procédé selon la revendication 1, **caractérisé en ce que**, pour la fabrication de 1-[6-(morpholin-4-yl)pyrimidin-4-yl]-4-(1H-1,2,3-triazol-1-yl)-1H-pyrazol-5-ol (I - forme énol) ou de 2-[6-(morpholin-4-yl)pyrimidin-4-yl]-4-(1H-1,2,3-triazol-1-yl)-1,2-dihydro-3H-pyrazol-3-one (I - forme céto), de l'acrylate de méthyl-(2E/Z)-3-(diméthylamino)-2-(1H-1,2,3-triazol-1-yle) (VII-Me) ou de l'acrylate d'éthyl-(2E/Z)-3-(diméthylamino)-2-(1H-1,2,3-triazol-1-yle) (VII-Et) est mis en réaction avec de la 4-(6-hydrazinopyrimidin-4-yl)morpholine (XI) en présence d'acide trifluoroacétique dans un solvant inerte, puis ajout de triéthylamine, puis le composé de formule (I) est isolé.

3. Procédé selon la revendication 2, **caractérisé en ce que** le mélange réactionnel est maintenu à une température de 20 à 90 °C après l'ajout de triéthylamine.

4. Procédé selon l'une quelconque des revendications 1 à 3, **caractérisé en ce que** la purification du produit brut obtenu du composé de formule (I) a lieu avec de l'eau et un acide à un pH de 4 à 5,5.

5. Procédé selon l'une quelconque des revendications 1 à 4, **caractérisé en ce que** le composé de formule (I) ainsi obtenu est mis en réaction lors d'une étape ultérieure avec de l'hydroxyde de sodium ou de la soude aqueuse ou du méthylate de sodium ou de l'éthylate de sodium ou un sel de sodium en 1-[6-(morpholin-4-yl)pyrimidin-4-yl]-4-(1H-1,2,3-triazol-1-yl)-1H-pyrazol-5-olate de sodium (II).

6. Procédé selon l'une quelconque des revendications 1 à 4, dans lequel la 4-(6-hydrazinopyrimidin-4-yl)morpholine (XI) est fabriquée par mise en réaction, lors de la première étape, tout d'abord de 4,6-dichloropyrimidine (VIII) avec de l'hydrate d'hydrazine (XII - hydrate) dans un solvant, éventuellement en présence d'une base auxiliaire, et chauffage du mélange réactionnel obtenu sans isolement de la 4-chloro-6-hydrazinopyrimidine (XV) formée lors d'une deuxième étape après l'ajout de morpholine (IX) et d'une autre base auxiliaire, le composé de formule (XI) étant ensuite isolé par cristallisation.

7. Procédé selon la revendication 6, **caractérisé en ce que** la réaction est réalisée avec de la triéthylamine ou de l'éthyldiisopropylamine en tant que base auxiliaire.

8. Procédé selon l'une quelconque des revendications 6 ou 7, **caractérisé en ce que** la réaction est réalisée avec de l'hydrogénocarbonate de sodium, de l'hydrogénocarbonate de potassium, de l'hydroxyde de sodium ou de l'hydroxyde de potassium en tant qu'autre base auxiliaire.

9. Procédé selon l'une quelconque des revendications 1 à 4, dans lequel l'acrylate de méthyl-(2E/Z)-3-(diméthylamino)-2-(1H-1,2,3-triazol-1-yle) (VII-Me) ou l'acrylate d'éthyl-(2E/Z)-3-(diméthylamino)-2-(1H-1,2,3-triazol-1-yle) (VII-Et) est fabriqué par mise en réaction des composés acétate de méthyl-1H-1,2,3-triazol-1-yle (V-Me) et acétate de méthyl-2H-1,2,3-triazol-1-yle (VI-Me) ou des composés acétate d'éthyl-1H-1,2,3-triazol-1-yle (V-Et) et acétate d'éthyl-2H-1,2,3-triazol-1-yle (VI-Et), qui sont présents en un rapport d'au moins 6:1 les uns par rapport aux autres, avec du diméthylformamide-diméthylacétal (XIX-Me) ou du diméthylformamide-diéthylacétal (XIX-Et) dans un solvant inerte, puis cristallisation par refroidissement de la solution ou par élimination par distillation du solvant et ajout d'un deuxième solvant.

10. Procédé selon la revendication 9, dans lequel les composés acétate de méthyl-1H-1,2,3-triazol-1-yle (V-Me) et acétate de méthyl-2H-1,2,3-triazol-1-yle (VI-Me) ou les composés acétate d'éthyl-1H-1,2,3-triazol-1-yle (V-Et) et acétate d'éthyl-2H-1,2,3-triazol-1-yle (VI-Et) sont fabriqués par mise en réaction de 1,2,3-triazole (III) avec de l'ester méthylique de l'acide bromoacétique (IV-Me-Br) ou de l'ester éthylique de l'acide bromoacétique (IV-Et-Br) en présence d'éthyldiisopropylamine en tant que base dans un solvant dans une plage de température de 20 à 80 °C.
